# EUROPÄISCHE PATENTSCHRIFT

(11) **EP 4 243 667 B1**
(45) Veröffentlichungstag und Bekanntmachung des Hinweises auf die Patenterteilung: **18.03.2026**
(21) Anmeldenummer: 21805534.1
(22) Anmeldetag: 08.11.2021
(51) Int. Cl.: A61B 1/00, A61B 1/05, A61B 5/00, G02B 13/00

(54) **ENDOSKOPISCHE UND/ODER EXOSKOPISCHE BILDGEBUNGSVORRICHTUNG ZUR SPEKTRALEN BILDGEBUNG UND VERFAHREN ZU DEREN BETRIEB**
ENDOSCOPIC AND/OR EXOSCOPIC IMAGING DEVICE FOR SPECTRAL IMAGING, AND METHOD FOR THE OPERATION THEREOF
DISPOSITIF D'IMAGERIE ENDOSCOPIQUE ET/OU EXSCOPIQUE POUR IMAGERIE SPECTRALE, ET SON PROCÉDÉ DE FONCTIONNEMENT

(30) Priorität: 11.11.2020 DE 102020129739
(43) Veröffentlichungstag der Anmeldung: 20.09.2023
(73) Patentinhaber: Karl Storz SE & Co. KG, 78532 Tuttlingen (DE)
(72) Erfinder: BAUMANN, Harald, 78532 Tuttlingen (DE); FALLERT, Johannes, 78532 Tuttlingen (DE); GÖBEL, Werner, 78532 Tuttlingen (DE); IRION, Klaus, 78532 Tuttlingen (DE)
(86) Internationale Anmeldenummer: PCT/EP2021/080946
(87) Internationale Veröffentlichungsnummer: WO 2022/101137

(56) Entgegenhaltungen:
- DE-A1- 102014 115 738
- US-A1- 2010 079 587

## Beschreibung

### Stand der Technik

Die Erfindung betrifft eine endoskopische- und/oder exoskopische Bildgebungsvorrichtung gemäß dem Oberbegriff des Anspruchs 1.

Aus der DE 10 2014 115 738 A1 ist bereits eine endoskopische Bildgebungsvorrichtung zur spektralen Bildgebung bekannt. Diese umfasst einen Schaft und einen in dem Schaft angeordneten Bildgebungskanal. Der Bildgebungskanal weist einen Bildgebungszweig auf, welcher wenigstens einen ersten spektralselektiven Strahlteiler umfasst, der spektral selektiv eine optische Abbildung in eine spektrale Teilabbildung eines Spektralbereichs und eine weitere spektrale Teilabbildung eines weiteren Spektralbereichs aufteilt. Dabei ist der Spektralbereich von dem weiteren Spektralbereich verschieden. Der Bildgebungszweig umfasst zur Erfassung der spektralen Teilabbildung einen Bildsensor. Zur Weiterleitung der weiteren Teilabbildung umfasst der Bildgebungszweig ferner eine Relaisoptik.

Aus US2010/0079587 A1 ist zudem ein Endoskop bekannt, das an einem distalen Ende seines Schafts zwei oder drei hintereinander angeordnete Strahlteiler sowie dazwischenliegende Abschwächungselemente aufweist, wobei den Strahlteilern jeweils ein Bildsensor zugeordnet ist, und wobei die Bildsensoren in unterschiedlichen Wellenlängenbereichen arbeiten. Bilddaten werden dann durch elektrische Leitungen vom distalen Ende des Schafts zu dessen proximalem Ende geleitet.

Die Aufgabe der Erfindung besteht insbesondere darin, unter Berücksichtigung einer kompakten Bauweise eine gattungsgemäße Vorrichtung mit verbesserten Eigenschaften hinsichtlich einer multispektralen und insbesondere einer hyperspektralen Bildgebung bereitzustellen. Die Aufgabe wird erfindungsgemäß durch die Merkmale des Anspruchs 1 gelöst, während vorteilhafte Ausgestaltungen und Weiterbildungen der Erfindung den Unteransprüchen entnommen werden können.

### Vorteile der Erfindung

Die Erfindung geht aus von einer endoskopischen- und/oder exoskopischen Bildgebungsvorrichtung zur multispektralen und insbesondere hyperspektralen, Bildgebung eines Untersuchungsgebiets; mit wenigstens einem Schaft und mit wenigstens einem zumindest teilweise, vorzugsweise zumindest zu einem Großteil und besonders bevorzugt vollständig, in dem Schaft angeordneten Bildgebungskanal, welcher wenigstens einen ersten Bildgebungszweig aufweist, welcher wenigstens einen ersten spektralselektiven Strahlteiler umfasst, der spektral selektiv eine optische Abbildung des zu überprüfenden Untersuchungsgebiets in wenigstens eine erste spektrale Teilabbildung eines ersten Spektralbereichs und wenigstens eine weitere erste spektrale Teilabbildung eines weiteren ersten Spektralbereichs aufteilt, wobei der erste Spektralbereich von dem weiteren ersten Spektralbereich verschieden ist, und der erste Bildgebungszweig wenigstens zur Erfassung der ersten spektralen Teilabbildung einen ersten Bildsensor umfasst, sowie der erste Bildgebungszweig zur Weiterleitung der weiteren ersten Teilabbildung wenigstens eine erste Relaisoptik umfasst, die ein Stablinsenpaar umfasst.

Es wird vorgeschlagen, dass der Bildgebungskanal wenigstens einen zweiten Bildgebungszweig aufweist, welcher wenigstens einen zweiten spektralselektiven Strahlteiler umfasst, der spektral selektiv die weitere erste Teilabbildung in wenigstens eine zweite spektrale Teilabbildung eines zweiten Spektralbereichs und wenigstens eine weitere zweite spektrale Teilabbildung eines weiteren zweiten Spektralbereichs aufteilt, wobei der zweite Spektralbereich von dem weiteren zweiten Spektralbereich verschieden ist, und der zweite Bildgebungszweig wenigstens zur Erfassung der zweiten spektralen Teilabbildung wenigstens einen zweiten Bildsensor umfasst, sowie der zweite Bildgebungszweig zur Weiterleitung der weiteren zweiten Teilabbildung wenigstens eine zweite Relaisoptik umfasst, die ein Stablinsenpaar umfasst, wobei die erste Relaisoptik des ersten Bildgebungszweigs und die zweite Relaisoptik des zweiten Bildgebungszweigs hintereinander angeordnet sind, so dass eine Bildebene der ersten Relaisoptik mit einem Objektebene der zweiten Relaisoptik identisch ist.

Hierdurch kann vorteilhaft ein Bauraum einer multispektralen und insbesondere hyperspektralen endoskopischen- und/oder exoskopischen Bildgebungsvorrichtung verringert werden. Dies gelingt insbesondere durch die Anordnung der Bildsensoren der verschiedenen Bildgebungszweige. Gerade für den endoskopischen Einsatz der Bildgebungsvorrichtung ist dies von Interesse, da bei dieser Anwendung nur der für einen chirurgisch minimalinvasiven Eingriff unbedingt notwendige Bauraum genutzt werden kann. Weiter vorteilhaft kann eine spektrale Auflösung erhöht werden, welche durch die Anzahl der verwendeten Bildgebungszweige vervielfacht werden kann. Weiter vorteilhaft kann durch die Verwendung zweier Bildkanäle und derer Sensoren eine spektrale Darstellung in Echtzeit mittels eines Videos, insbesondere mittels eines Stereovideos, erzielt werden. Unter einer "endoskopischen- und/oder exoskopischen Bildgebungsvorrichtung" soll insbesondere ein, vorzugsweise funktionsfähiger Bestandteil, insbesondere eine Unterbaugruppe und/oder eine Konstruktions- und/oder eine Funktionskomponente eines Endoskops und/oder Exoskops verstanden werden. Die endoskopische- und/oder exoskopische Bildgebungsvorrichtung kann das Endoskop und/oder das Exoskop zumindest teilweise, vorzugsweise zumindest zu einem Großteil und besonders bevorzugt vollständig ausbilden. Die "endoskopische Bildgebungsvorrichtung" ist insbesondere dazu eingerichtet, zumindest teilweise und vorzugsweise zumindest zu einem Großteil in eine künstliche Kavität, wie beispielsweise ein Gehäuse, und/oder natürlichen Kavität, wie beispielsweise einen Hohlraum in einem Körperorgan oder im Gewebe, eingeführt zu werden, um diese von innen zu begutachten. Die "exoskopische Bildgebungsvorrichtung" ist insbesondere dazu eingerichtet, außerhalb einer künstlichen Kavität, wie beispielsweise ein Gehäuse, und/oder einer natürlichen Kavität, wie beispielsweise einen Hohlraum in einem Körperorgan oder im Gewebe, angeordnet zu werden, um diese von außen zu begutachten. Unter "eingerichtet" soll insbesondere speziell programmiert, vorgesehen, ausgebildet, ausgelegt und/oder ausgestattet verstanden werden. Darunter, dass ein Bauteil zu einer bestimmten Funktion eingerichtet ist, soll insbesondere verstanden werden, dass das Bauteil diese bestimmte Funktion in zumindest einem Anwendungs- und/oder Betriebszustand erfüllt und/oder ausführt. Unter dem Ausdruck "zumindest zu einem Großteil" soll dabei insbesondere zumindest zu 50 %, vorzugsweiser zumindest zu 70 %, bevorzugt zumindest zu 90 % und ganz besonders bevorzugt vollständig verstanden werden und zwar insbesondere bezüglich eines Volumens und/oder einer Masse eines Bauteils. Die endoskopische- und/oder exoskopische Bilderfassungsvorrichtung weist wenigstens einen proximalen Abschnitt und einen distalen Abschnitt auf. Der distale Abschnitt ist insbesondere dazu ausgebildet, in einem Betriebszustand in einer zu untersuchenden Kavität eingeführt zu werden. Unter "distal" soll insbesondere bei einer Bedienung einem Patienten zugewandt und/oder einem Bediener abgewandt verstanden werden. Insbesondere ist proximal das Gegenteil von distal. Unter "proximal" soll insbesondere bei einer Bedienung einem Patienten abgewandt und/oder einem Bediener zugewandt verstanden werden. Der proximale Abschnitt ist insbesondere dazu ausgebildet, in einem Betriebszustand außerhalb der zu untersuchenden Kavität angeordnet zu sein. Der Schaft kann beispielsweise den proximalen Abschnitt wenigstens teilweise ausbilden. Der Schaft ist insbesondere als ein längliches Bauteil ausgebildet. Der Schaft ist vorzugsweise als ein starrer Schaft ausgebildet. Alternativ könnte der Schaft flexibel ausgebildet sein. Unter einem "länglichen Bauteil" soll insbesondere ein Bauteil verstanden werden, dessen Haupterstreckung zumindest um einen Faktor fünf, vorzugsweise zumindest um einen Faktor zehn und besonders bevorzugt zumindest um einen Faktor zwanzig größer ist als eine größte Erstreckung des Bauteils senkrecht zu dessen Haupterstreckung, also insbesondere einem Durchmesser des Bauteils. Unter einer "Haupterstreckung" eines Bauteils, soll insbesondere dessen längste Erstreckung entlang dessen Haupterstreckungsrichtung verstanden werden. Unter einer "Haupterstreckungsrichtung" eines Bauteils soll insbesondere eine Richtung verstanden werden, welche parallel zu einer längsten Kante eines kleinsten gedachten Quaders verläuft, welcher das Bauteil gerade noch vollständig umschließt.

Unter "spektraler Bildgebung" soll insbesondere eine Bildgebung verstanden werden, bei welcher wenigstens ein spektrales Abbild eines Untersuchungsgebiets erzeugt wird, welche Informationen wenigstens zweier räumlicher Dimensionen und wenigstens einer spektralen Dimension umfasst. Das spektrale Abbild, kann somit durch einen Datenwürfel, welcher wenigstens Zeileneinträge und Spalteneinträge, welche eine räumliche Information umfassen sowie Ebeneneinträge, welche spektrale Informationen umfassen, aufweist, beschrieben werden. Ferner ist denkbar, dass die spektrale Abbildung auch höher dimensioniert sein kann, wie beispielsweise in Form eines Datenhyperwürfels oder Datentesserakt, und drei räumliche Dimensionen, eine zeitliche Dimension und eine spektrale Dimension umfassen kann. In diesem Fall würde die spektrale Abbildung in Form eines 5-dimensionalen Datenhyperwürfels vorliegen. Die spektralen Informationen einer spektralen Abbildung liegen insbesondere anhand von Spektralkennwerten vor. Anhand der Spektralkennwerte kann mittels der Bildgebungsvorrichtung auf wenigstens eine Art und/oder wenigstens eine Eigenschaft, wie beispielweise eine Gewebeart und/oder eine Gewebeeigenschaft, des zu betrachtenden Untersuchungsgebiets geschlossen werden. Die Spektralkennwerte dienen insbesondere als Stützstellen, welche mit hinterlegten für eine bestimmte Art und/oder Eigenschaft des Untersuchungsgebiets charakteristischen Kennwerten abgeglichen werden können, um die wenigstens eine Art und/oder die wenigstens eine Eigenschaft des Untersuchungsgebiets zu bestimmen. Unter "multispektraler Bildgebung" soll eine spektrale Bildgebung verstanden werden, bei welcher eine Anzahl von wenigstens 3, vorzugsweise wenigstens 5 und besonders bevorzugt wenigstens 9 zueinander spektral versetzter Spektralkennwerte erfasst wird. Unter "hyperspektraler Bildgebung" soll insbesondere eine spektrale Bildgebung verstanden werden, bei welcher eine Anzahl von wenigstens 10, vorzugsweise von wenigstens 40 und besonders bevorzugt von wenigstens 120 zueinander spektral versetzter Spektralkennwerte erfasst werden. Die erfassten Spektralkennwerte liegen dabei insbesondere in einem Wellenlängenbereich ab wenigstens 10 nm, vorzugsweise ab wenigstens 150 nm und besonders bevorzugt ab wenigstens 300 nm, und/oder bis höchsten 3000 nm, vorzugsweise bis höchstens 2000 nm und besonders bevorzugt bis höchstens 1000 nm, also beispielsweise auch innerhalb des ultravioletten und infraroten Wellenlängenbereichs.

Unter einem "Bildgebungskanal" soll insbesondere ein optischer Übertragungsweg verstanden werden, welcher zur Erfassung der spektralen Abbildung beiträgt. Ein Bildgebungszweig eines Bildgebungskanals ist insbesondere ein Abschnitt des Bildgebungskanals, entlang welchem dieser eine Verzweigung eines optischen Übertragungswegs bzw. Strahlengangs aufweist. Unter einer "Relaisoptik" eines Bildgebungszweigs soll insbesondere eine Optik verstanden werden, welche eine Abbildung entlang eines optischen Übertragungswegs verlängert und ein Zwischenbild der Abbildung wenigstens einmalig umkehrt entlang des Übertragungswegs umkehrt. Die Relaisoptik des Bildgebungszweigs kann eine oder mehreren Linsen, vorzugsweise wenigstens eine Gradientenlinse, sowie Achromaten, Apochromaten oder dergleichen, in etwa zur Korrektur chromatischer Abbildungsfehler, umfassen.

Bei einem spektral selektiven Strahlteiler handelt es sich insbesondere um ein optisches Bauteil, welches eine optische Abbildung in wenigstens eine erste spektrale Teilabbildung eines ersten Spektralbereichs und wenigstens eine weitere erste spektrale Teilabbildung eines weiteren ersten Spektralbereichs aufteilt, wobei jedoch die räumlichen Informationen der Abbildung auch in den jeweiligen Teilabbildungen erhalten bleibt. Der Strahlteiler kann als ein Reflexionsstrahlteiler, Interferrenzstrahlteiler, Strahlenteilerwürfel, dichroitischer Spiegel oder dergleichen ausgebildet sein. Darunter, dass "ein Spektralbereich von einem weiteren Spektralbereich" verschieden ist, soll insbesondere verstanden werden, dass die Spektralbereiche sich spektral wenigstens teilweise, vorzugsweise wenigstens zu einem Großteil und besonders bevorzugt vollständig nicht gegenseitig überlappen. Die Spektralbereiche können insbesondere verschiedene spektrale Breiten aufweisen. Der erste spektrale Teilbereich erstreckt sich vorzugsweise über den sichtbaren Spektralbereich. Der zweite Spektralbereich erstreckt sich besonders bevorzugt über den nahinfraroten Spektralbereich.

Unter einem "Bildsensor" soll insbesondere ein Sensor verstanden werden, welcher in einer 2-dimensionalen Matrix angeordnete Sensorpixel aufweist. Bei dem Bildsensor kann es sich um einen CMOS-Sensor, einen CCD-Sensor oder dergleichen handeln. Der Bildsensor ist insbesondere dazu eingerichtet, Bilder mit einer Bildrate von wenigstens 15, vorzugsweise wenigstens 30 und besonders bevorzugt wenigstens 60 Bildern pro Sekunde aufzunehmen, wodurch spektrale Informationen als Video bzw. in Echtzeit aufgenommen und/oder wiedergegeben werden können. Der Bildsensor weist insbesondere wenigstens eine Spektralempfindlichkeit auf, welche die wellenlängenabhängige Empfindlichkeit des Bildsensors definiert und welche durch eine Empfindlichkeitskennlinie beschreibbar ist. Die Spektralempfindlichkeit ist insbesondere abhängig von einer Quanteneffizienz eines Sensorpixels des Bildsensors und/oder eines dem Sensorpixel zugeordneten Filterspektrums eines Sensorfilters des Bildsensors. Somit kann durch Verwendung verschiedener Sensorfilter oder Sensorpixel eine Spektralempfindlichkeit variiert werden. Gelangt in etwa Licht einer Teilabbildung eines Spektralbereichs dieser Teilabbildung zum Bildsensor, so registriert der Bildsensor dieses in Abhängigkeit des jeweiligen Spektralbereichs der Teilabbildung und der Spektralempfindlichkeit des Bildsensors selbst. Ein gesuchter Spektralkennwert ergibt sich aus der Überlagerung des übertragenen Lichts der Teilabbildung des Spektralbereichs der Teilabbildung und der Spektralempfindlichkeit des Sensors.

Wie oben beschrieben ist eine Anzahl der mit der Bildgebungsvorrichtung bestimmbaren Stützstellen skalierbar mit der Anzahl der verschiedener spektral selektiven Strahlteiler der Bildgebungszweige. Um vorteilhaft eine spektrale Auflösung weiter zu erhöhen und trotzdem eine kompakte Bauweise bei zuhalten wird vorgeschlagen, dass der Bildgebungskanal wenigstens einen dritten Bildgebungszweig aufweist, welcher wenigstens einen dritten spektralselektiven Strahlteiler umfasst, der spektral selektiv die weitere zweite Teilabbildung in wenigstens eine dritte spektrale Teilabbildung eines dritten Spektralbereichs und wenigstens eine weitere dritte spektrale Teilabbildung eines weiteren dritten Spektralbereichs aufteilt, wobei der dritte Spektralbereich von dem weiteren dritten Spektralbereich verschieden ist, und der dritte Bildgebungszweig wenigstens zur Erfassung der dritten spektralen Teilabbildung wenigstens einen dritten Bildsensor umfasst, sowie der dritte Bildgebungszweig zur Weiterleitung der weiteren dritten Teilabbildung wenigstens eine dritte Relaisoptik umfasst, wobei die zweite Relaisoptik des zweiten Bildgebungszweigs und die dritte Relaisoptik des dritten Bildgebungszweigs hintereinander angeordnet sind, so dass eine Bildebenen der zweiten Relaisoptik mit einer Objektebene der dritten Relaisoptik identisch ist. Der dritte Spektralbereich erstreckt sich vorzugsweise über den infraroten Spektralbereich. Besonders vorteilhaft kann die spektrale Auflösung weiter gesteigert werden, indem eine Vielzahl von Bildgebungszweigen gemäß der Anordnung des ersten und des zweiten Bildkanals kaskadiert hintereinander angeordnet sind.

Um vorteilhaft einen Bauraum weiter zu verringern und eine platzsparende Anordnung zu erzielen wird ferner vorgeschlagen, dass wenigstens ein spektralselektiver Strahlteiler und/oder wenigstens ein Bildsensor der Bildgebungszweige zwischen zwei Relaisoptiken verschiedener Bildgebungszweige angeordnet sind/ist. Insbesondere sind der wenigstens eine spektralselektiver Strahlteiler und/oder der wenigstens eine Bildsensor der Bildgebungszweige zwischen einer Relaisoptik eines der Bildgebungszweige und dessen Bildebene angeordnet. Ferner ist vorzugsweise jeweils wenigstens der eine Bildsensor dem wenigstens einen Strahlteiler des Bildgebungszweigs zugeordnet und diesem optisch nachgeschaltet. Denkbar ist, dass weitere optische Bauteile, wie beispielsweise Linsen, Prismen oder dergleichen, zwischen dem Strahlteiler und dem Strahlteiler zugeordneten Bildsensor angeordnet sein können. Vorteilhaft ist der Bildsensor unmittelbar auf den Strahlteiler folgend angeordnet. Insbesondere könnte der Bildsensor mit dem Strahlteiler kraft- und/oder formschlüssig verbunden sein. Unter "kraft- und/oder formschlüssig" soll insbesondere verstanden werden, dass eine Haltekraft zwischen zwei Bauteilen vorzugsweise durch einen geometrischen Eingriff der Bauteile ineinander und/oder eine Reibkraft zwischen den Bauteilen übertragen wird. Ferner könnten der Bildsensor und der Strahlteiler auch stoffschlüssig miteinander verbunden sein. Ferner könnten diese auch einstückig miteinander verbunden sein. Unter "einstückig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Bevorzugt sind jeweilige spektralselektive Strahlteiler und/oder Bildsensoren benachbarter Bildgebungszweige zwischen zwei Relaisoptiken der benachbarten Bildgebungszweige angeordnet. Besonders bevorzugt gilt dies für alle Strahlteiler und/oder Bildsensoren der kaskadenartig angeordneten Bildgebungszweige.

Weiterhin wird vorgeschlagen, dass wenigstens einer der Bildgebungszweige wenigstens ein weiteres optisches Bauteil umfassen, dessen Brechungsindex und/oder dessen Material zumindest im Wesentlichen mit einem Brechungsindex und/oder einem Material eines spektralselektiven Strahlteilers des jeweiligen Bildgebungszweigs übereinstimmt, und welches zwischen zwei Relaisoptiken verschiedener Bildgebungszweige angeordnet ist. Es können vorteilhaft chromatische Abbildungsfehler, welche ansonsten eine Genauigkeit einer spektralen Analyse als auch einer optischen Abbildung verringern würden, ausgeglichen oder vermieden werden. Weiter vorteilhaft kann trotz zusätzlicher Bauteile ein benötigter Bauraum geringgehalten werden. Unter dem Ausdruck "zumindest im Wesentlichen" in Bezug auf eine Eigenschaft, einen Zahlenwert oder dergleichen, soll insbesondere die Eigenschaft, der Zahlenwert oder dergleichen verstanden werden und zwar vorzugsweise unter Berücksichtigung einer Abweichung von höchstens 15%, vorzugsweise von höchstens 10 % und besonders bevorzugt von höchstens 5 %. Insbesondere ist das wenigstens eine weitere optische Bauteil der Bildgebungszweige zwischen einer Relaisoptik eines der Bildgebungszweiges und dessen Objektebene angeordnet. Das weitere optische Bauteil ist vorzugsweise als ein Glaswürfel ausgebildet. Bevorzugt weist jeder der Bildgebungszweige ein entsprechendes weiteres optisches Bauteil auf, welches auch entsprechend zwischen benachbarten Bildgebungszweigen angeordnet ist.

Um durch eine symmetrische Anordnung Abbildungsfehler zu vermeiden, wird zudem vorgeschlagen, dass der spektralselektive Strahlteiler und das weitere optische Bauteil spiegelsymmetrisch zu der miteinander identischen Bildebene und Objektebene zweier insbesondere benachbarter Relaisoptiken verschiedener Bildgebungszweige angeordnet sind. Vorzugsweise sind jeweilige spektralselektive Strahlteiler und jeweilige weitere optische Bauteile spiegelsymmetrisch zu der miteinander identischen Bildebene und Objektebene zweier Relaisoptiken benachbarter Bildgebungszweige angeordnet.

Weiterhin wird vorgeschlagen, dass der spektralselektive Strahlteiler und das weitere optische Bauteil miteinander zumindest teilweise einstückig verbunden oder ausgebildet sind. Es kann vorteilhaft vermieden werden, dass in Grenzbereichen zwischen diesen Bauteilen weitere Abbildungsfehler generiert werden. Weiter vorteilhaft können Reflektionen oder Streuungen vermieden werden.

Es wird zudem vorgeschlagen, dass wenigstens einer der Spektralbereiche der Teilabbildungen der Bildgebungszweige kürzere Wellenlängen umfasst, als wenigstens ein anderer Spektralbereich der Teilabbildungen der Bildgebungszweige. Es kann vorteilhaft eine spektrale Aufteilung der Abbildung in die Teilabbildungen verbessert werden. Besonders bevorzugt sind alle Spektralbereiche der Teilabbildungen voneinander verschieden. Insbesondere nehmen die Wellenlängen der Spektralbereich der Teilabbildungen stufenweise **für** jede der hintereinander angeordneten Bildgebungszweige in proximaler oder distaler Richtung zu.

Ferner wird vorgeschlagen, dass wenigstens ein Bildsensor oder wenigstens zwei Bildsensoren der Bildgebungszweige eine Sensorebene aufweist/aufweisen, welche zumindest im Wesentlichen parallel zu einer Mittelachse des Schafts angeordnet ist/sind. Es kann vorteilhaft eine besonders kompakte Anordnung der Bildsensoren erzielt werden. Unter einer "Sensorebene" soll insbesondere eine Haupterstreckungsebene des Sensors verstanden werden. In der Sensorebene sind insbesondere die Sensorpixel in einer Matrix angeordnet. Vorzugsweise sind alle Sensorebenen aller Bildsensoren der Bildgebungszweige zumindest im Wesentlichen parallel zu der Mittelachse angeordnet. Besonders bevorzugt liegen alle Bildsensoren in einer einzigen Ebene, welche zumindest im Wesentlichen parallel zur Mittelachse des Schafts liegt. Unter "im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2° aufweist.

Es wird außerdem vorgeschlagen, dass der Bildgebungskanal wenigstens eine Leiterplatte aufweist, auf welcher wenigstens ein Bildsensor oder wenigstens zwei Bildsensoren der Bildgebungszweige und besonders bevorzugt alle der Bildsensoren der Bildgebungszweige, angeordnet ist/sind. Es kann vorteilhaft eine platzsparende und einfache Anordnung der Bildsensoren erzielt werden, um diese elektrisch zu kontaktieren. Die Leiterplatte definiert insbesondere eine Ebene, welche zumindest im Wesentlichen parallel zur Mittelachse des Schafts liegt. Die Leiterplatte könnte als eine flexible Leiterplatte ausgebildet sein. Vorzugsweise ist die Leiterplatte als ein PCB ausgebildet. Die Leiterplatte ist insbesondere in dem Schaft angeordnet. Ferner ist denkbar, dass die Leiterplatte in den Schaft integriert ist.

Es wird vorgeschlagen, dass wenigstens ein Bildsensor oder wenigstens zwei Bildsensoren der Bildgebungszweige wenigstens eine erste Spektralempfindlichkeit und wenigstens eine zweite Spektralempfindlichkeit, sowie insbesondere wenigstens eine dritte Spektralempfindlichkeit, aufweist/aufweisen, in Abhängigkeit welcher dieser/diese eine Teilabbildung der Bildgebungszweige erfasst/erfassen. Es kann vorteilhaft eine spektrale Auflösung weiter erhöht werden. Diese kann durch die Anzahl verschiedener Spektralempfindlichkeiten der Bildsensoren weiter erhöht werden. Dass ein Bildsensor verschiedene Spektralempfindlichkeiten aufweist, kann dadurch erzielt werden, dass die Sensorpixel verschiedenen Sensorfiltern zugeordnet werden. Der Bildsensor kann beispielsweise als ein RGB-Sensor ausgebildet werden, welcher eine erste Spektralempfindlichkeit im blauen sichtbaren Spektrum, eine zweite Spektralempfindlichkeit im grünen sichtbaren Spektrum und eine dritte Spektralempfindlichkeit im roten sichtbaren Spektrum umfasst.

Es ist denkbar, dass alle Bildsensoren der Bildgebungszweige gleiche Spektralempfindlichkeiten aufweisen. Eine spektrale Auflösung der Bildgebungsvorrichtung lässt sich jedoch mit einer Anzahl verschiedener Spektralempfindlichkeiten der verschiedenen Bildsensoren weiter erhöhen. Es wird deshalb vorgeschlagen, dass zumindest ein Bildsensor der Bildgebungszweige wenigstens eine Spektralempfindlichkeit aufweist, welche von einer Spektralempfindlichkeit eines anderen Bildsensors der Bildgebungszweige verschieden ist. Vorzugsweise sind die Spektralempfindlichkeiten der aller Bildsensoren voneinander verschieden.

Es wird vorgeschlagen, dass der Bildergebungskanal zur Erfassung wenigstens einer der Teilabbildungen der Bildgebungszweige wenigstens eine Kamera aufweist, welche in einem proximalen und insbesondere extrakorporalen Endabschnitts des Schafts angeordnet ist. Es kann vorteilhaft zusätzliche Bildeigenschaften mittels der separaten Kamera erfasst werden. Unter einem "Endabschnitt" soll insbesondere ein Abschnitt verstanden werden, welcher sich ausgehend von einem Ende eines Bauteils in Richtung der Mitte des Bauteils erstreckt. Um eine spektrale Auflösung besonders zu erhöhen wird vorgeschlagen, dass die Kamera als eine multispektrale und/oder hyperspektrale Kamera ausgebildet ist. Besonders bevorzugt arbeitet die Kamera nach dem spatial-scanning-Prinzip es sind jedoch auch andere einem Fachmann bekannte Bildgebungsprinzipien denkbar.

Es wird weiter vorgeschlagen, dass die Bildgebungsvorrichtung wenigstens eine Lichtquelle umfasst, welche in wenigstens einem Betriebszustand ein abzubildendes Untersuchungsgebiet mit wenigstens einem Beleuchtungsspektrum beleuchtet. Da das Beleuchtungsspektrum bekannt ist, kann dies bei einer Auswertung mit einbezogen werden, wodurch ein Informationsgehalt der Auswertung verbessert werden kann. Das Licht der Lichtquelle trifft insbesondere auf das abzubildende Untersuchungsgebiet und wird dort wenigstens teilweise absorbiert, transmittiert oder reflektiert. Der absorbierte Anteil des Lichts der Lichtquelle kann von dem Untersuchungsgebiet zumindest teilweise in Form von Fluoreszenz und/oder Phosphoreszenz wieder abgestrahlt werden. Mittels der Bildgebungsvorrichtung kann wenigstens ein reflektierter Anteil des Lichts der Lichtquelle und/oder eine von dem Untersuchungsgebiet abgegebene Fluoreszenz und/oder Phosphoreszenz zur Abbildung des Untersuchungsgebiets genutzt werden. Auch ist denkbar, den transmittierten Anteil mit der vorliegenden Bildgebungsvorrichtung zu erfassen. Bei der Lichtquelle kann es sich um eine Breitbandlichtquelle, eine Weißlichtlichtquelle, eine Laserlichtquelle oder dergleichen handeln. Die Lichtquelle kann wenigstens eine und vorzugsweise mehrere Lichtelemente aufweisen, wie beispielsweise LEDs, OLEDs, Laser oder dergleichen. Das Beleuchtungsspektrum der Lichtquelle könnte zeitlich variierbar sein, z.B. durch Abschalten und/oder Hinzuschalten verschiedener Lichtelemente. Die Lichtquelle umfasst, wenigsten ein Lichtelement, welches Weißlicht erzeugt. Unter "Weißlicht" soll insbesondere polychromatische Licht verstanden werden, welche zumindest im sichtbaren Spektralbereich zumindest im Wesentlich dem Tageslichtspektrum entspricht. Der sichtbare Spektralbereich erstreckt sich dabei insbesondere zwischen 380 nm und 780 nm.

Es wird vorgeschlagen, dass die Lichtquelle Beleuchtungslicht abstrahlt, welches ein Beleuchtungsspektrum umfasst, welches breiter ist, als wenigstens ein Spektralbereich der Teilabbildungen der Bildgebungszweige. Es kann vorteilhaft mit einer Lichtquelle, der für die spektrale Untersuchung interessante Bereich des Spektrums abgedeckt werden. Die Lichtquelle kann insbesondere wenigstens zwei und vorzugsweise mehrere Beleuchtungslichter abstrahlen, welche voneinander verschieden sind. Dies können sich durch ihr spektrales Abstrahlverhalten unterscheiden. Die Lichtquelle weist insbesondere wenigstens ein Lichtquellenelement auf. Vorzugsweise umfasst die Lichtquelle wenigstens zwei und besonders bevorzugt mehrere Lichtquellenelemente. Bei dem Lichtquellenelement kann es sich um eine LED, OLED, Laserdiode oder dergleichen handeln. Die Lichtquellenelemente können sich durch ein von Ihnen bereitgestelltes Beleuchtungslicht voneinander unterscheiden.

Alternativ oder zusätzlich wird vorgeschlagen, dass die Lichtquelle Beleuchtungslicht abstrahlt, welches wenigstens ein Beleuchtungsspektrum umfasst, welches innerhalb wenigstens eines Spektralbereichs der Teilabbildungen der Bildgebungszweige liegt. Es kann vorteilhaft eine besonders scharfe spektrale Auflösung erzielt werden.

Es wird weiterhin vorgeschlagen, dass die Bildgebungsvorrichtung wenigstens einen weiteren Bildgebungskanal umfasst. Es kann vorteilhaft ein Informationsgehalt der spektralen Bildgebung erhöht werden, da insbesondere durch die Kombination der Informationen des Bildgebungskanals und des weiteren Bildgebungskanals auch stereoskopische Informationen zusätzlich zu den spektralen Informationen gewonnen werden können. Der Bildgebungskanal und der weitere Bildgebungskanal unterscheiden sich zumindest durch einen mittels des jeweiligen Bildgebungskanals registriert waren Ausschnitts eines mittels der endoskopischen- und/oder exoskopischen Bildgebungsvorrichtung Untersuchungsgebiet. Der Bildgebungskanal und der weitere Bildgebungskanal sind insbesondere zueinander versetzt angeordnet und zwar vorzugsweise zumindest im Wesentlichen senkrecht zu Mittelachsen der jeweiligen Bildgebungskanäle.

Ferner kann der Bildgebungskanal und der Weiterbildungsgang unterschiedliche Blickrichtungen aufweisen, beispielsweise können die Bildgebungskanäle zueinander in Klick angeordnet sein. Vorzugsweise sind der Bildgebungskanal und der weitere Bildgebungskanal jedoch zumindest im Wesentlichen parallel zueinander angeordnet. Insbesondere könnte die Bildgebungsvorrichtung einer Mehrzahl von Bildgebungskanälen aufweisen, welche sich wenigstens durch eine Blickrichtung voneinander unterscheiden könnten und oder zueinander versetzt angeordnet sein können.

Zudem wird vorgeschlagen, dass sich die Bildkanäle wenigstens durch eine Spektralselektivität wenigstens eines spektralselektiven Strahlteilers ihrer Bildgebungszweige und/oder durch eine Spektralempfindlichkeit wenigstens eines Bildsensors ihrer Bildzweige voneinander unterscheiden. Es kann vorteilhaft eine spektrale Auflösung weiter erhöht werden. Die durch die verschiedenen Bildkanäle gewonnenen Bilder und spektralen Informationen kann ein stereoskopisches und spektral aufgelöstes Bild erzeugt werden. Insbesondere können die spektralen Informationen zu den stereoskopischen Informationen gematcht werden.

Es wird vorgeschlagen, dass der Bildgebungskanal und der weitere Bildgebungskanal zueinander spiegelsymmetrisch angeordnet sind. Es kann vorteilhaft eine besonders baulich kompakte Anordnung der Bildgebungskanäle zueinander erzielt werden. Insbesondere sind die Bildsensoren des Bildgebungskanals und des weiteren Bildgebungskanals auf derselben Leiterplatte angeordnet und zwar vorzugsweise spiegelsymmetrisch zueinander.

In einem weiteren Aspekt der Erfindung, welcher allein aber auch in Kombination mit dem Vorhergehenden betrachtet werden kann, wird vorgeschlagen, dass die Bildgebungsvorrichtung zumindest wenigstens einen weiteren Bildgebungskanal aufweist, welcher frei von einer Relaisoptik ist. Hierdurch kann vorteilhaft eine stereo Bildgebungsvorrichtung bereitgestellt werden, welche sich auch für Endoskope mit geringen Schaftdurchmessern eignet. Insbesondere umfasst der weitere Bildgebungskanal wenigstens ein weiteres Objektiv und ein dem Objektiv lichtstromaufwärts folgend angeordneten Bildsensor auf. Ferner kann der weitere Bildgebungszweig einen Spiegel und/oder einen Strahlteiler aufweisen, welcher durch das Objektiv eintretendes Licht wenigstens teilweise, vorzugsweise zumindest zu einem Großteil und besonders bevorzugt vollständig umlenkt. Bei dem Strahlteiler handelt es sich insbesondere um einen spektralselektiven Strahlteiler.

Ferner wird vorgeschlagen, dass der Schaft wenigstens abschnittsweise im Wesentlichen von dem Bildgebungskanal ausgefüllt ist. Es kann vorteilhaft ein Schaftdurchmesser verringert werden, da dieser lediglich auf die Abmessungen des Bildgebungskanals und nicht des weiteren Bildgebungskanals angepasst werden kann. Darunter, dass der Schaft abschnittsweise von dem Bildgebungskanal ausgefüllt ist, soll insbesondere verstanden werden, dass der wenigstens einen Abschnitt, beispielsweise einen mittleren Abschnitt, zwischen distalem und proximalen Endabschnitten aufweist, in welchen der Schaft von dem Bildgebungskanal wenigstens zu 50%, vorzugsweise wenigstens zu 65% und besonders bevorzugt wenigstens zu 80% seines Volumens durch den Bildgebungskanal ausgefüllt ist.

Um gleichzeitig verschiedene Bildgebungsmethoden nutzen zu können wird ferner vorgeschlagen, dass der Bildgebungskanal zu einer multispektralen bzw. hyperspektralen Bildgebung eingerichtet ist und der weitere Bildgebungskanal zu einer Weißlichtbildgebung eingerichtet ist.

Des Weiteren wird vorgeschlagen, dass die Bildgebungsvorrichtung eine Steuereinrichtung, welche dazu eingerichtet ist, wenigstens ein mit dem Bildgebungskanal aufgenommenes multispektrales und/oder hyperspektrales Bild mit wenigstens einem von dem weiteren Bildgebungskanal aufgenommenen Weißlichtbild zu matchen. Hierdurch kann digital eine multispektrale und/oder hyperspektrale Stereobildgebung erzielt werden. Ein solches Matchen der Weißlichtbilds und des Multispektral- bzw. Hyperspektralbildes kann mittels eines in der Steuereinrichtung hinterlegten Matching-Algorithmus erfolgen. Dieser kann beispielsweise auf der Methodik der Bildstabilisierung, des Feature-Trackings, des Marker-Trackings oder dergleichen beruhen.

Ferner wird vorgeschlagen, dass in einem distalen Endabschnitt des Schafts wenigstens ein Temperatursensor angeordnet ist, welcher zu einer temperaturabhängigen Ansteuerung zumindest des Bildgebungskanals eingerichtet ist. Es kann vorteilhaft eine durch distale Elektronik erzeugte Temperaturanstieg überwacht werden und durch gezieltes Aktivieren und/oder Deaktivieren der Elektronik, wie beispielsweise der Bildsensoren das Überschreiten einer Grenztemperatur, welche zu Verletzungen an einem Patienten und/oder zu Beschädigungen an der Bildgebungsvorrichtung führen könnte vermieden werden. Ferner kann der Temperatursensor alternativ oder zusätzlich zur temperaturabhängigen Ansteuerung des weiteren Bildgebungskanals eingerichtet sein. Dazu ist der Temperatursensor in etwa mit der Steuereinrichtung gekoppelt. Insbesondere ist durch die einzuhaltende Grenztemperatur auch eine für die Lichtquelle einzuhaltende Bestrahlungszeit bestimmt.

Es wird weiter vorgeschlagen, dass in einem distalen Endabschnitt des Schafts wenigstens ein Bewegungssensor angeordnet ist, welcher zu einer bewegungsabhängigen Ansteuerung zumindest des Bildgebungskanals eingerichtet ist. Der Bewegungssensor ist dazu eingerichtet eine konstante Position der Bildgebungsvorrichtung während einer Aufnahme einer Abbildung zu registrieren. Insbesondere für den Fall, dass eine Bewegung registriert wird, wird eine Meldung an die Steuereinrichtung übergeben und eine Aufnahme gestoppt. Ferner ist es denkbar, dass eine Bewegung mittels des Bewegungssensors aufgezeichnet wird und eine Bildgebung basierend auf dieser erfassten Bewegung korrigiert wird.

Ferner wird ein Endoskop und/oder Exoskop mit wenigstens einer solchen endoskopischen- und/oder exoskopischen Bildgebungsvorrichtung vorgeschlagen. Hierdurch kann vorteilhaft ein Bauraum einer multispektralen und insbesondere hyperspektralen endoskopischen- und/oder exoskopischen Bildgebungsvorrichtung verringert werden.

Die Bildgebungsvorrichtung, das Endoskop und/oder Exoskop und/oder ein Verfahren zum Betrieb sollen/soll hierbei nicht auf die oben beschriebene Anwendung und Ausführungsform beschränkt sein. Insbesondere kann die Bildgebungsvorrichtung, das Endoskop und/oder Exoskop und/oder das Verfahren zum Betrieb zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Falls von einem bestimmten Bauteil mehr als ein Exemplar vorhanden ist, ist nur eines davon in den Figuren und in der Beschreibung mit einem Bezugszeichen versehen. Die Beschreibung dieses Exemplars kann entsprechend auf die anderen Exemplare von dem Bauteil übertragen werden.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer Bildgebungsvorrichtung in einer perspektivischen Ansicht,
- Fig. 2: eine schematische Darstellung eines Teils der Bildgebungsvorrichtung in einer Schnittansicht,
- Fig. 3: einen Aufbau einer Kamera der Bildgebungsvorrichtung in einer schematischen Darstellung,
- Fig. 4: ein schematisches Diagramm, welches die spektralen Eigenschaften einer Lichtquelle der Bildgebungsvorrichtung darstellt,
- Fig. 5: ein schematisches Diagramm, welches beispielhafte spektrale Eigenschaften eines mittels der Bildgebungsvorrichtung abzubildenden Untersuchungsgebiets darstellt,
- Fig. 6: ein schematisches Diagramm, in welchem optische Eigenschaften von Strahlteilern der Bildgebungsvorrichtung idealisiert dargestellt sind,
- Fig. 7: ein schematisches Diagramm, in welchem weitere optische Eigenschaften von Strahlteilern der Bildgebungsvorrichtung idealisiert dargestellt sind,
- Fig. 8: ein schematisches Diagramm, in welchem Detektionseigenschaften der Bildsensoren der Bildgebungsvorrichtung dargestellt sind,
- Fig. 9: ein schematisches Diagramm, in welchem Spektralempfindlichkeiten der Bildsensoren dargestellt sind,
- Fig. 10: ein schematisches Diagramm, in welchem Spektralempfindlichkeiten der Bildsensoren dargestellt sind,
- Fig. 11: ein schematisches Diagramm, in welchem Spektralempfindlichkeiten der Bildsensoren dargestellt sind,
- Fig. 12: einen schematischen Ablaufplan eines beispielhaften Verfahrens zum Betrieb der endoskopischen Bildgebungsvorrichtung,
- Fig. 13: eine schematische Darstellung einer exoskopischen Bildgebungsvorrichtung in einer perspektivischen Ansicht,
- Fig. 14: ein schematisches Diagramm mit optische Eigenschaften einer alternativen Bildgebungsvorrichtung,
- Fig. 15: ein schematisches Diagramm mit optische Eigenschaften einer weiteren Ausgestaltung einer weiteren endoskopischen und/oder exoskopische Bildgebungsvorrichtung
- Fig. 16: eine schematische Darstellung einer alternativen endoskopischen und/oder exoskopischen Bildgebungsvorrichtung in einer Schnittansicht,
- Fig. 17: eine schematische Darstellung einer alternativen endoskopischen und/oder exoskopischen Bildgebungsvorrichtung in einer Schnittansicht,
- Fig. 18: eine schematische Darstellung einer weiteren alternativen endoskopischen und/oder exoskopischen Bildgebungsvorrichtung in einer Schnittansicht und,
- Fig. 19: eine schematische Darstellung einer spektralen Aufteilung einer mit der Bildgebungsvorrichtung aufgenommenen optischen Abbildung in Teilabbildungen.

### Beschreibung der Ausführungsbeispiele

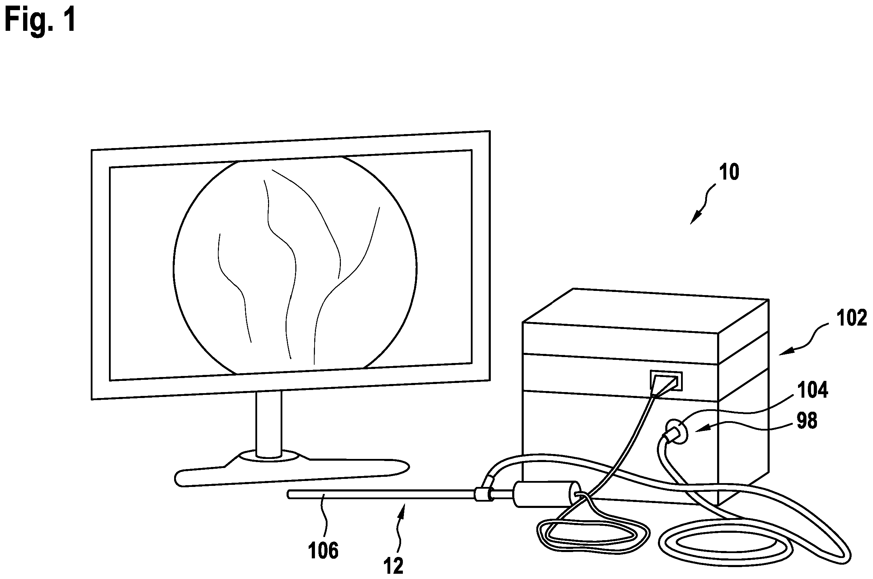

Fig. 1 zeigt eine schematische Darstellung einer endoskopischen Bildgebungsvorrichtung 10 in einer perspektivischen Ansicht. Die endoskopische Bildgebungsvorrichtung 10 bildet ein Endoskop 12 vollständig aus. Alternativ könnte die endoskopische Bildgebungsvorrichtung nur einen funktionsfähigen Bestandteil eines Endoskops ausbilden. Alternativ oder zusätzlich kann es sich bei der hierin beschriebenen Bildgebungsvorrichtung auch um eine exoskopische Bildgebungsvorrichtung handeln. Eine solche exoskopische Bildgebungsvorrichtung könnte ein Exoskop vollständig oder nur einen funktionsfähigen Bestandteil des Exoskops ausbilden. Auch ist die Eignung derselben Vorrichtung sowohl für den exoskopischen als auch den endoskopischen Einsatz denkbar. Insbesondere ist die folgende Beschreibung auch auf eine exoskopische Bildgebungsvorrichtung 10 anwendbar.

Die endoskopische Bildgebungsvorrichtung 10 ist zur spektralen Bildgebung eingerichtet. Im vorliegenden Fall ist die endoskopische Bildgebungsvorrichtung 10 wenigstens zur multispektralen Bildgebung eingerichtet. Ferner kann die endoskopische Bildgebungsvorrichtung 10 sogar zu einer hyperspektralen Bildgebung eingerichtet sein. Die endoskopische Bildgebungsvorrichtung 10 ist dazu eingerichtet, in einem Betriebszustand wenigstens ein Untersuchungsgebiet abzubilden. Bei dem Untersuchungsgebiet handelt es sich im vorliegenden Fall um Gewebe, beispielsweise eines Körperteils. Das Untersuchungsgebiet kann im inneren eines Körpers liegen. Dazu ist die endoskopische Bildgebungsvorrichtung 10 dazu eingerichtet, zumindest teilweise in eine Kavität angeordnet zu werden.

Die Bildgebungsvorrichtung 10 weist eine Steuereinrichtung 102 auf. Die Steuereinrichtung 102 ist dazu eingerichtet, weitere funktionelle Komponenten der endoskopischen Bildgebungsvorrichtung 10 anzusteuern. Die Steuereinrichtung 102 weist eine Steuerelektronik (nicht dargestellt) auf. Die Steuerelektronik umfasst einen Prozessor. Ferner besitzt die Steuerelektronik einen Speicher. Auf dem Speicher ist ein Programm zum Betrieb der endoskopischen Bildgebungsvorrichtung 10 hinterlegt. Der Prozessor ist dazu eingerichtet, das Programm zum Betrieb der endoskopischen Bildgebungsvorrichtung 10 auszuführen. Die Steuereinrichtung 102 ist im vorliegenden Fall in einem gemeinsamen Gehäuse mit weiteren Komponenten der endoskopischen Bildgebungsvorrichtung 10 angeordnet. Alternativ könnte die Steuereinrichtung jedoch als ein separates Steuergerät ausbilden.

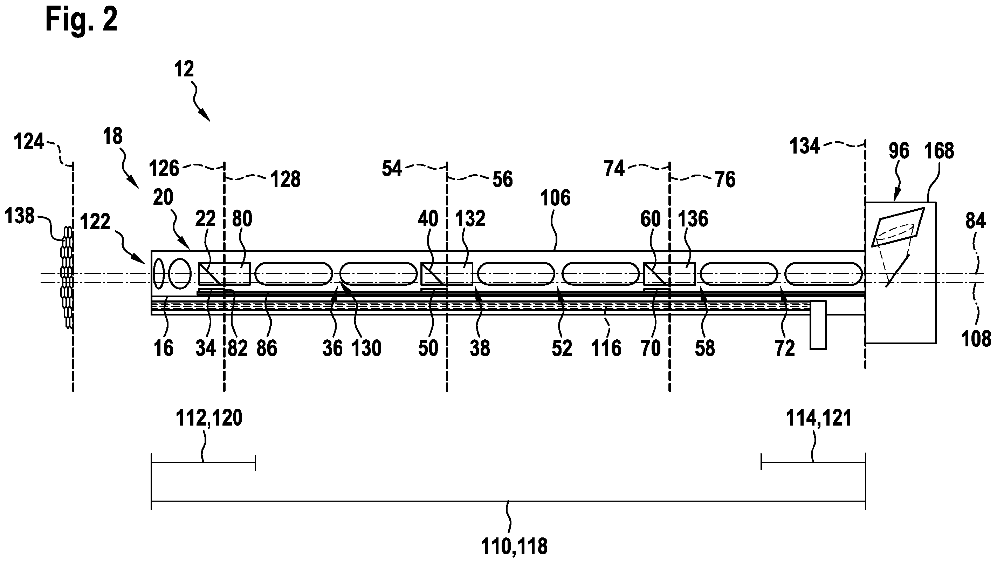

Die endoskopische Bildgebungsvorrichtung 10 weist einen Überschafft 106 auf. Der Überschafft 106 ist als ein längliches Bauteil ausgebildet. Der Überschafft 106 weist eine Mittelachse 108 auf (vgl. Fig. 2). Entlang der Mittelachse 108 verläuft eine Haupterstreckung 110 des Überschafft 106. Ein Querschnitt des Überschaffts 106 senkrecht zu dessen Mittelachse 108 weist einen Durchmesser auf, welcher Wesentlich kleiner als die Haupterstreckung 110 des Überschafft 106. Der Durchmesser ist wenigstens um den Faktor 2 kleiner als die Haupterstreckung 110. Im vorliegenden Fall ist der Durchmesser sogar wenigstens um den Faktor 5 kleiner als die Haupterstreckung 110. Der Überschafft 106 ist röhrenförmig ausgebildet. Der Überschaft 106 ist aus Metall ausgebildet. Beispielsweise kann der Überschaft aus Stahl, Titan, Chrom oder dergleichen ausgebildet sein. Der Überschafft 106 weist einen distalen Endabschnitt 112 auf. Ferner weist der Überschaft 106 einen proximalen Endabschnitt 114 auf. Der Überschafft 106 ist dazu eingerichtet, in einem Betriebszustand über eine natürlich oder künstlich geschaffene Öffnung, wie beispielsweise eine Körperöffnung, in eine Kavität eingeführt zu werden.

Fig. 2 zeigt eine schematische Darstellung eines Teils der endoskopischen Bildgebungsvorrichtung 10 in einer Schnittansicht. Die endoskopische Bildgebungsvorrichtung 10 weist wenigstens einen Schaft 16 auf. Der Schaft 16 ist in dem Überschafft 106 angeordnet. Der Schaft 16 ist im vorliegenden Fall als ein längliches Bauteil ausgebildet. Der Schaft 16 weist eine Mittelachse 84 auf. Entlang der Mittelachse 84 verläuft eine Haupterstreckung 118 des Schafts 16. Ein Querschnitt des Schafts 16 senkrecht zu dessen Mittelachse 84 weist einen Durchmesser auf, welcher wesentlich kleiner als die Haupterstreckung 118 des Schafts 16 ist. Der Durchmesser ist wenigstens um den Faktor 2 kleiner als die Haupterstreckung 118. Im vorliegenden Fall ist der Durchmesser sogar wenigstens um den Faktor 5 kleiner als die Haupterstreckung 118. Der Schaft 16 ist röhrenförmig ausgebildet. Der Schaft 16 ist aus Metall ausgebildet. Beispielsweise kann der Schaft 16 aus Stahl, Titan, Chrom oder dergleichen ausgebildet sein. Der Schaft 16 weist einen distalen Endabschnitt 120 auf. Ferner weist der Schaft 16 einen proximalen Endabschnitt 121 auf.

Die endoskopische Bildgebungsvorrichtung 10 weist wenigstens einen Bildgebungskanal 18 auf. Der Bildgebungskanal 18 ist zumindest teilweise in dem Schaft 16 angeordnet. Der Bildgebungskanal 18 ist dazu eingerichtet, eine optische Abbildung 24 eines abzubildenden Untersuchungsgebiets 138 zumindest teilweise entlang dem Schaft 16 zu übertragen.

Der Bildgebungskanal 18 weist ein Objektiv 122 auf. Das Objektiv 122 ist wenigstens teilweise in dem Schaft 16 angeordnet. Das Objektiv 122 ist im distalen Endabschnitt 120 des Schafts 16 angeordnet. Das Objektiv 122 ist zur Erzeugung einer optischen Abbildung 24 des Untersuchungsgebiets eingerichtet. Das Objektiv 122 weist eine Objektebene 124 auf. In einem Betriebszustand befindet sich das Untersuchungsgebiet in der Objektebene 124. Das Objektiv 122 weist ferner eine Bildebene 126 auf. Das Objektiv 122 bildet eine optische Abbildung 24 des sich in der Objektebene 124 befindlichen Untersuchungsgebiet auf die Bildebene 126 ab.

Der Bildgebungskanal 18 weist einen ersten Bildgebungszweig 20 auf. Der erste Bildgebungszweig 20 ist wenigstens teilweise in dem Schaft 16 angeordnet. Im vorliegenden Fall ist der erste Bildgebungszweig 20 vollständig in dem Schaft 16 angeordnet. Der erste Bildgebungszweig 20 ist dem Objektiv 122 lichtstromaufwärts nachgeschaltet.

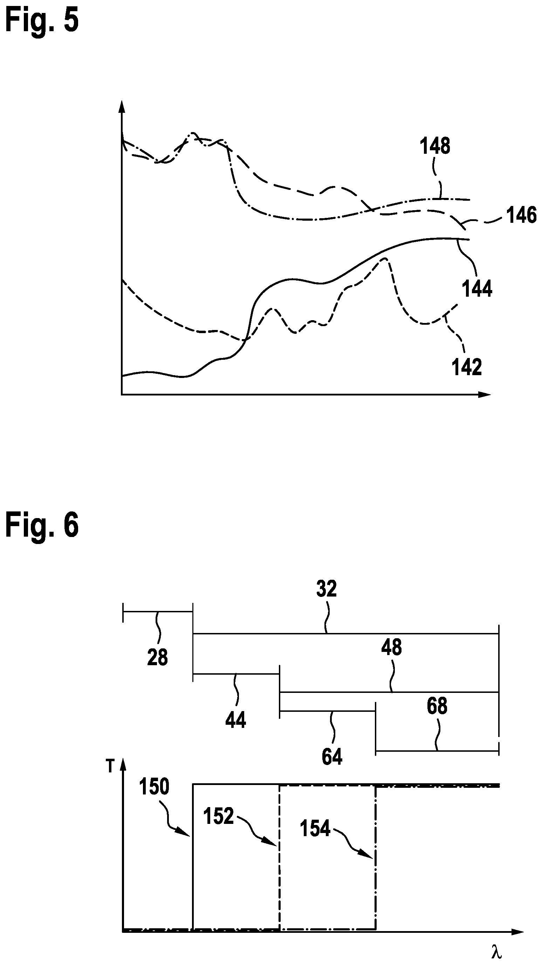

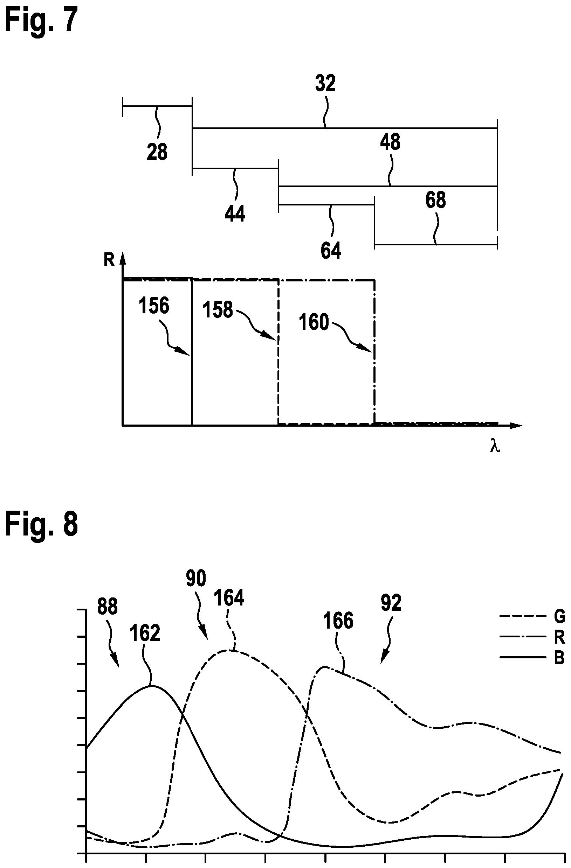

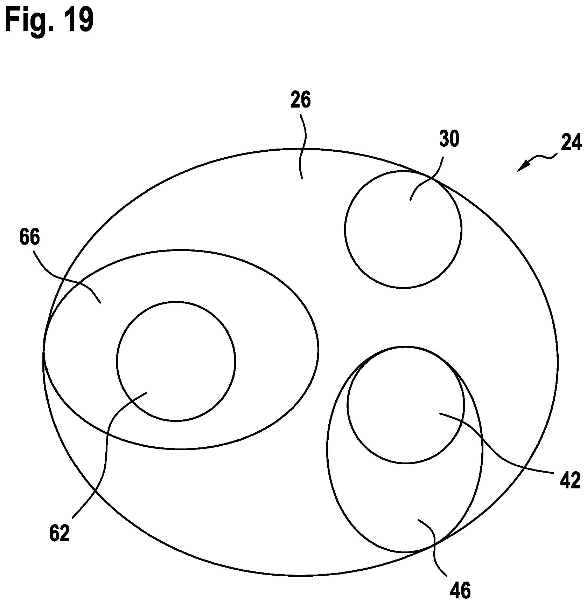

Der erste Bildgebungszweig 20 weist wenigstens einen ersten spektralselektiven Strahlteiler 22 auf. Der erste spektralselektive Strahlteiler 22 teilt die optische Abbildung 24 des Untersuchungsgebiets in wenigstens eine erste spektrale Teilabbildung 26 eines ersten Spektralbereichs 28 und wenigstens eine weitere erste spektrale Teilabbildung 30 eines weiteren ersten Spektralbereichs 32 auf. Die Teilabbildungen 26, 30 und die Spektralbereiche 28, 32 sind in den Figure 6 und 7 dargestellt, welche im Folgenden noch näher beschrieben werden. Die optische Abbildung 24 sowie die Teilabbildungen sind in Fig. 19 dargestellt. Der erste Spektralbereich 28 ist von dem weiteren ersten Spektralbereich 32 verschieden. Der erste spektralselektive Strahlteiler 22 lenkt die erste spektrale Teilabbildung 26 ab, im vorliegenden Fall um 90°. Die weitere erste spektrale Teilabbildung 30 durchläuft den ersten spektralen Strahlteiler 22 in gleichbleibender Richtung. Denkbar ist, dass die Spektralbereiche durch Steuerung des Strahlteilers variable sein könnten. Beispielsweise könnte ein Winkel einer Reflexionsebene des Strahlteilers relativ zum einfallenden Lichtstrahl bzw. zur Mittelachse 84 variiert werden, um dies zu erzielen. Im vorliegenden Fall ist ein Winkel der Reflexionsebene des ersten spektralen Strahlteilers 22 jedoch fest gewählt. Der Winkel beträgt 45° zum einfallenden Lichtstrahl bzw. zur Mittelachse 84 des Schafts 16.

Der erste Bildgebungszweig 20 weist wenigstens einen ersten Bildsensor 34 auf. Der erste Bildsensor 34 ist zur Erfassung der ersten spektralen Teilabbildung 26 eingerichtet. Durch die Ablenkung des ersten spektralselektiven Strahlteilers 22 ist die erste spektrale Teilabbildung 26 auf den ersten Bildsensor 34 gerichtet. Der erste Bildsensor 34 liegt in einer Bildebene der ersten spektralen Teilabbildung 26. Somit erfolgt eine scharfe Abbildung der ersten spektralen Teilabbildung 26 auf den ersten Bildsensor 34. Der erste Bildsensor 34 weist eine Sensorebene 82 auf. Die Sensorebene 82 ist zumindest im Wesentlichen parallel zu der Mittelachse 84 des Schafts 16 angeordnet.

Zur Weiterleitung der weiteren ersten Teilabbildung 30 weist der erste Bildgebungszweig 20 wenigstens eine erste Relaisoptik 36 auf. Die erste Relaisoptik 36 ist lichtstromaufwärts dem ersten selektiven Strahlenteiler 22 nachgeschaltet. Die erste Relaisoptik 36 bildet die weitere erste Teilabbildung 30 aus einer Objektebene 128 der ersten Relaisoptik in eine Bildebene 54 ab. Das Objektiv 122 und die erste Relaisoptik 36 sind hintereinander angeordnet, so dass die Bildebene 126 des Objektivs 122 mit der Objektebene 128 der ersten Relaisoptik 36 identisch ist. Derart leitet die erste Relaisoptik 36 die weitere erste Teilabbildung 30 maßstabsgetreu weiter. Die erste Relaisoptik 36 weist ein Stablinsenpaar 130 auf. Ferner weist die erste Relaisoptik 36 zwei Achromaten, Apochromaten oder dergleichen auf (nicht dargestellt). Diese können zwischen dem Stablinsenpaar angeordnet sein. Mittels dieser können chromatische Abbildungsfehler, welche sich aufgrund der im Folgenden beschriebenen kaskadenartigen Anordnung mehrere Bildgebungszweige weiter verstärken, verringert werden.

Der erste spektralselektiver Strahlteiler 22 ist zwischen dem Objektiv 122 und der ersten Relaisoptik 36 des ersten Bildgebungszweigs 20 angeordnet. Genauer gesagt ist der erste spektralselektive Strahlteiler 22 zwischen dem Objektiv 122 und der Bildebene 126 des Objektivs 122 bzw. der Objektebene 128 der ersten Relaisoptik 36 angeordnet.

Der erste Bildsensor 34 ist zwischen dem Objektiv 122 und der ersten Relaisoptik 36 des ersten Bildgebungszweigs 20 angeordnet. Genauer gesagt ist der erste Bildsensor 34 zwischen dem Objektiv 122 und der Bildebene 126 des Objektivs 122 bzw. der Objektebene 128 der ersten Relaisoptik 36 angeordnet. Der erste Bildsensor 34 ist dem ersten spektralselektiven Strahlteiler 22 lichtstromaufwärts nachgeschaltet. Dabei sind der erste spektralselektive Strahlteiler 22 und der erste Bildsensor 34 so angeordnet, dass ein optischer Weg durch den ersten spektralselektiven Strahlteiler 22 zum ersten Bildsensor 34 gleich einem optischen Weg durch den ersten spektralselektiven Strahlteiler 22 zur Bildebene 126 des Objektivs 122 bzw. der Objektebene 128 der ersten Relaisoptik 36 entspricht.

Der erste Bildgebungszweig 20 weist wenigstens ein erstes weiteres optisches Bauteil 80 auf. Ein Brechungsindex und/oder ein Material des ersten weiteren optischen Bauteils 80 stimmt zumindest im Wesentlichen mit einem Brechungsindex und/oder einem Material des ersten spektralselektiven Strahlteilers 22 überein. Das erste weitere optische Bauteil 80 ist zwischen dem Objektiv 122 und der ersten Relaisoptik 36 angeordnet. Genauer gesagt ist das erste weitere optische Bauteil 80 zwischen dem ersten spektralselektiven Strahlteiler 22 und der ersten Relaisoptik 36 angeordnet. Das weitere optische Bauteil 80 ist derart angeordnet, dass die Bildebene 126 des Objektivs 122 bzw. die Objektebene 128 der ersten Relaisoptik 36 zwischen dem ersten weiteren optischen Bauteil 80 und dem ersten spektralselektiven Strahlteiler 22 liegt. Der erste spektralselektive Strahlteiler 22 und das erste weitere optische Bauteil 80 sind spiegelsymmetrisch zu der miteinander identischen Bildebene 126 des Objektivs 122 und der Objektebene 128 der ersten Relaisoptik 36 angeordnet. Im vorliegenden Fall ist das erste weiter optische Bauteil 80 einstückig mit dem ersten spektralselektiven Strahlteiler 22 ausgebildet oder verbunden. Beispielsweise könnten der Strahlteiler und das weitere optische Bauteil miteinander verklebt oder verkittet sein. Ferner ist denkbar, dass der Strahlteiler und das weitere optische Bauteil zumindest teilweise aus einem Rohling heraus einstückig gefertigt sind. Alternativ könnten das weitere optische Bauteil und der Strahlteiler auch separat voneinander ausgebildet sein.

Im vorliegenden Fall weist der Bildgebungskanal 18 mehrere Bildgebungszweige 20, 36, 58 auf und zwar beispielsweise im vorliegenden Fall einen ersten Bildgebungszweig 20, einen zweiten Bildgebungszweig 38 und einen dritten Bildgebungszweig 58. Die Bildgebungszweige 20, 38, 58 sind im vorliegenden Fall bis auf ihre spektralen Eigenschaften im Wesentlichen identisch ausgebildet. Deshalb wird im Folgenden nur auf die spezielle Anordnung der Bildgebungszweige 20, 38, 58 und deren Bauteile zueinander eingegangen. Soweit nicht anders ausgeführt, ist eine Beschreibung der Bauteile des Bildgebungszweigs 20 auf Bauteile der Bildgebungszweige 38, 58 übertragbar. Zwar weist der Bildgebungskanal 18 in dieser Ausgestaltung insgesamt drei Bildgsbungszweige auf, jedoch ist es denkbar, dass die Anzahl für einen Fachmann naheliegend angepasst und insbesondere vergrößert werden kann, um eine Anzahl von mit den Bildgebungszweigen 20, 38, 58 ermittelbaren Spektralkennwerten zu erhöhen. Dadurch könnte eine spektrale Auflösung der Vorrichtung verbessert werden.

Der Bildgebungskanal 18 weist wenigstens einen zweiten Bildgebungszweig 38 auf. Der zweite Bildgebungszweig 38 ist zumindest teilweise in dem Schaft 16 angeordnet. Im vorliegenden Fall ist der zweite Bildgebungszweig 38 vollständig in dem Schaft 16 angeordnet. Ferner ist der zweite Bildgebungszweig 38 dem ersten Bildgebungszweig 20 lichtstromaufwärts nachgeschaltet. Ferner ist der erste Bildgebungszweig 20 zwischen dem Objektiv 122 und dem zweiten Bildgebungszweig 38 angeordnet.

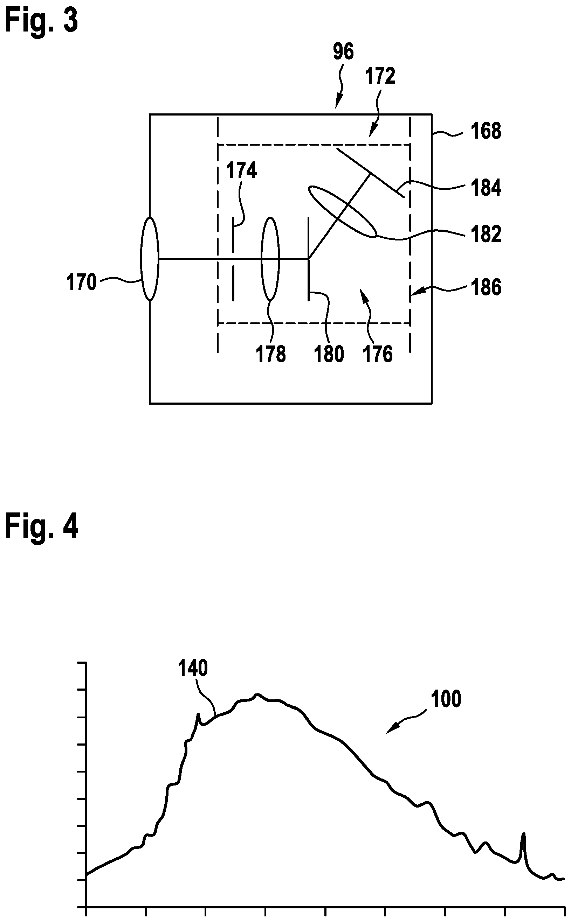

Der zweite Bildgebungszweig 38 weist wenigstens einen zweiten spektralselektiven Strahlteiler 40 auf. Der zweite spektralselektive Strahlteiler 40 ist seinen optischen Eigenschaften von dem ersten spektralselektierenden Starhalteiler 22 verschieden ausgebildet. Der zweite spektralselektive Strahlteiler 40 teilt spektral selektiv die weitere erste Teilabbildung 30 in wenigstens eine zweite spektrale Teilabbildung 42 eines zweiten Spektralbereichs 44 und wenigstens eine weitere zweite spektrale Teilabbildung 46 eines weiteren zweiten Spektralbereichs 48 auf. Der zweite Spektralbereich 44 ist von dem weiteren zweiten Spektralbereich 48 verschieden (vgl. Fig. 3, 4 und 5). Dabei lenkt der zweite spektralselektive Strahlteiler 40 die zweite spektrale Teilabbildung 42 ab, im vorliegenden Fall um 90°. Die weitere zweite spektrale Teilabbildung 46 durchläuft den zweiten spektralselektiven Strahlteiler 40 mit gleichbleibender Richtung.

Der zweite Bildgebungszweig 38 weist wenigstens einen zweiten Bildsensor 50 auf. Der zweite Bildsensor 50 ist zur Erfassung der zweiten spektralen Teilabbildung 42 eingerichtet. Durch die Ablenkung des zweiten spektralselektiven Strahlteilers 40 ist die zweite spektrale Teilabbildung 42 auf den zweiten Bildsensor 50 gerichtet. Der zweite Bildsensor 50 liegt dabei in einer Bildebene der ersten spektralen Teilabbildung 26. Der zweite Bildsensor 50 weist eine Sensorebene 82 auf. Die Sensorebene 82 ist zumindest im Wesentlichen parallel zu der Mittelachse 84 des Schafts 16 angeordnet.

Zur Weiterleitung der weiteren zweiten Teilabbildung 46 weist der zweite Bildgebungszweig 38 wenigstens eine zweite Relaisoptik 52 auf. Die zweite Relaisoptik 52 ist lichtstromaufwärts dem zweiten selektiven Strahlenteiler 40 nachgeschaltet. Die zweite Relaisoptik 52 besitzt eine Objektebene 56. Ferner besitzt die zweite Relaisoptik 52 eine Bildebene 74. Die erste Relaisoptik 36 und die zweite Relaisoptik 52 sind hintereinander angeordnet, so dass die Bildebene 54 der ersten Relaisoptik 36 mit der Objektebene 56 der zweiten Relaisoptik 52 identisch ist. Derart leitet die zweite Relaisoptik 52 die weitere zweite Teilabbildung 30 maßstabsgetreu weiter.

Der zweite spektralselektiver Strahlteiler 40 ist zwischen der ersten Relaisoptik 36 und der zweiten Relaisoptik 52 des zweiten Bildgebungszweigs 38 angeordnet. Genauer gesagt ist der zweite spektralselektive Strahlteiler 40 zwischen der ersten Relaisoptik 36 und der Bildebene 54 der ersten Relaisoptik 36 bzw. der Objektebene 56 der zweiten Relaisoptik 52 angeordnet.

Der zweite Bildsensor 50 ist zwischen der ersten Relaisoptik 36 und zweiten Relaisoptik 52 des zweiten Bildgebungszweigs 38 angeordnet. Genauer gesagt ist der zweite Bildsensor 50 zwischen der ersten Relaisoptik 36 und der Bildebene 54 der ersten Relaisoptik 36 bzw. der Objektebene 56 der zweiten Relaisoptik 52 angeordnet. Der zweite Bildsensor 50 ist dem zweiten spektralselektiven Strahlteiler 40 lichtstromaufwärts nachgeschaltet. Dabei sind der zweite spektralselektive Strahlteiler 40 und der zweite Bildsensor 50 so angeordnet, dass ein optischer Weg durch den zweiten spektralselektiven Strahlteiler 40 zum zweiten Bildsensor 50 einem optischen Weg durch den zweiten spektralselektiven Strahlteiler 40 zur Bildebene 54 der ersten Relaisoptik 36 bzw. der Objektebene 56 der zweiten Relaisoptik 52 entspricht.

Der zweite Bildgebungszweig 38 weist wenigstens ein zweites weiteres optisches Bauteil 132 auf. Ein Brechungsindex und/oder ein Material des zweiten weiteren optischen Bauteils 132 stimmt zumindest im Wesentlichen mit einem Brechungsindex und/oder einem Material des zweiten spektralselektiven Strahlteilers 40 überein. Das zweite weitere optischen Bauteil 132 ist zwischen der ersten Relaisoptik 36 und der zweiten Relaisoptik 52 angeordnet. Genauer gesagt ist das zweite weitere optische Bauteil 132 zwischen dem zweiten Strahlteiler 40 und der zweiten Relaisoptik 52 angeordnet. Das weitere zweite optische Bauteil 132 ist derart angeordnet, dass die Bildebene 54 des ersten Relaisoptik 36 bzw. die Objektebene 56 der zweiten Relaisoptik 52 zwischen dem zweiten weiteren optischen Bauteil 132 und dem zweiten spektralselektiven Strahlteiler 40 liegt. Der zweite spektralselektive Strahlteiler 40 und das zweite weitere optische Bauteil sind spiegelsymmetrisch zu der miteinander identischen Bildebene 54 der ersten Relaisoptik 36 und Objektebene 56 der zweiten Relaisoptik 52 angeordnet. Im vorliegenden Fall ist das zweite weitere optische Bauteil 132 zumindest teilweise einstückig mit dem zweiten Strahlteiler 40 ausgebildet oder verbunden.

Der Bildgebungskanal 18 weist wenigstens einen dritten Bildgebungszweig 58 auf. Der dritten Bildgebungszweig 58 ist zumindest teilweise in dem Schaft 16 angeordnet. Im vorliegenden Fall ist der dritte Bildgebungszweig 58 vollständig in dem Schaft 16 angeordnet. Der dritte Bildgebungszweig 58 ist dem zweiten Bildgebungszweig 38 lichtstromaufwärts nachgeschaltet. In anderen Worten ist der zweite Bildgebungszweig 38 zwischen dem ersten Bildgebungszweig 20 und dem dritten Bildgebungszweig 58 angeordnet.

Der dritte Bildgebungszweig 58 weist wenigstens einen dritten spektralselektiven Strahlteiler 60 auf. Der dritte spektralselektive Strahlteiler 60 ist seinen optischen Eigenschaften von dem ersten spektralselektierenden Strahlteiler 20 verschieden ausgebildet. Ferner ist der dritte spektralselektive Strahlteiler 60 in seinen optischen Eigenschaften von dem zweiten spektralselektierenden Strahlteiler 40 verschieden ausgebildet. Der dritten spektralselektiven Strahlteiler 60 teilt spektral selektiv die weitere zweite Teilabbildung 46 in wenigstens eine dritte spektrale Teilabbildung 62 eines dritten Spektralbereichs 64 und wenigstens eine weitere dritte spektrale Teilabbildung 66 eines weiteren dritten Spektralbereichs 68 auf. Der dritte Spektralbereich 64 ist von dem weiteren dritten Spektralbereich 68 verschieden.

Der dritte Bildgebungszweig 58 weist wenigstens einen dritten Bildsensor 70 auf. Der dritte Bildsensor 70 ist zur Erfassung der dritten spektralen Teilabbildung 62 eingerichtet. Durch die Ablenkung des zweiten spektralselektiven Strahlteilers 40 ist die dritte spektrale Teilabbildung 62 auf den dritten Bildsensor 70 gerichtet. Der dritte Bildsensor 70 liegt dabei in einer Bildebene der dritten spektralen Teilabbildung 62. Der dritte Bildsensor 70 weist eine Sensorebene 82 auf. Die Sensorebene 82 ist zumindest im Wesentlichen parallel zu der Mittelachse 84 des Schafts 16 angeordnet.

Zur Weiterleitung der weiteren dritten Teilabbildung 66 weist der dritte Bildgebungszweig 58 wenigstens eine dritte Relaisoptik 72 auf. Die dritte Relaisoptik 72 ist lichtstromaufwärts dem dritten selektiven Strahlenteiler 60 nachgeschaltet. Die dritte Relaisoptik 72 besitzt eine Objektebene 76. Ferner besitzt die dritte Relaisoptik 72 eine Bildebene 134. Die dritte Relaisoptik 72 und die zweite Relaisoptik 52 sind hintereinander angeordnet, so dass die Bildebene 74 der zweiten Relaisoptik 52 mit der Objektebene 76 der dritten Relaisoptik 72 identisch ist. Derart leitet die dritte Relaisoptik 72 die weitere dritte spektrale Teilabbildung 66 maßstabsgetreu weiter. Die dritte Relaisoptik 72 weist ein Stablinsenpaar 130 auf.

Der dritte spektralselektiver Strahlteiler 60 ist zwischen der zweiten Relaisoptik 52 und der dritten Relaisoptik 72 des dritten Bildgebungszweigs 58 angeordnet. Genauer gesagt ist der dritte spektralselektive Strahlteiler 60 zwischen der zweiten Relaisoptik 52 und der Bildebene 74 der zweiten Relaisoptik 52 bzw. der Objektebene 76 der dritten Relaisoptik 72 angeordnet.

Der dritte Bildsensor 70 ist zwischen der zweiten Relaisoptik 52 und der dritten Relaisoptik 72 des dritten Bildgebungszweigs 58 angeordnet. Genauer gesagt ist der dritte Bildsensor 70 zwischen der zweiten Relaisoptik 52 und der Bildebene 74 der zweiten Relaisoptik 52 bzw. der Objektebene 76 der dritten Relaisoptik 72 angeordnet. Der dritte Bildsensor 70 ist dem dritten spektralselektiven Strahlteiler 60 lichtstromaufwärts nachgeschaltet. Dabei sind der dritte spektralselektive Strahlteiler 60 und der dritte Bildsensor 70 so angeordnet, dass ein optischer Weg durch den dritten spektralselektiven Strahlteiler 60 zum dritten Bildsensor 70 gleich einem optischen Weg durch den dritten spektralselektiven Strahlteiler 60 zur Bildebene 74 der zweiten Relaisoptik 52 bzw. der Objektebene 76 der dritten Relaisoptik 72 entspricht.

Der dritte Bildgebungszweig 58 weist wenigstens ein drittes weiteres optisches Bauteil 136 auf. Ein Brechungsindex und/oder ein Material des dritten weiteren optischen Bauteils 136 stimmt zumindest im Wesentlichen mit einem Brechungsindex und/oder einem Material des dritten spektralselektiven Strahlteilers 60 überein. Das dritte weitere optische Bauteil 136 ist zwischen der zweiten Relaisoptik 52 und der dritten Relaisoptik 72 angeordnet. Genauer gesagt ist das dritte weitere optische Bauteil 136 zwischen dem dritten spektralselektiven Strahlteiler 60 und der dritten Relaisoptik 72 angeordnet. Das dritte weitere optische Bauteil 136 ist derart angeordnet, dass die Bildebene 74 der zweiten Relaisoptik 52 bzw. die Objektebene 76 der dritten Relaisoptik 72 zwischen dem dritten weiteren optischen Bauteil 136 und dem dritten spektralselektiven Strahlteiler 60 liegt. Der dritte spektralselektive Strahlteiler 60 und das dritte weitere optische Bauteil 136 sind spiegelsymmetrisch zu der miteinander identischen Bildebene und Objektebene der zweiten Relaisoptik 52 und der dritten Relaisoptik 72 angeordnet. Im vorliegenden Fall ist das zweite weitere optische Bauteil 132 zumindest teilweise einstückig mit dem zweiten Strahlteiler 40 ausgebildet oder verbunden.

Der Bildgebungskanal 18 weist wenigstens eine Leiterplatte 86 auf. Auf der Leiterplatte 86 ist wenigstens einer der Bildsensoren 34, 50, 70 angeordnet. Im vorliegenden Fall sind sogar alle der Bildsensoren 34, 50, 70 auf der Leiterplatte 86 angeordnet. Die Leiterplatte 86 ist als eine PCB ausgebildet. Alternativ kann es sich auch bei einer solchen Leiterplatte um einen Flexprint oder dergleichen handeln.

Die Bildgebungsvorrichtung 10 weist wenigstens eine Lichtquelle 98 auf. Die Lichtquelle 98 ist von der Steuereinrichtung 102 gesteuert. Die Lichtquelle 98 ist in einem separaten Gehäuse der Bildgebungsvorrichtung 10 angeordnet. Die Lichtquelle 98 strahlt in wenigstens einem Betriebszustand ein Beleuchtungslicht ab. Die Lichtquelle 98 weist wenigstens ein Leuchtelement 104 auf. Das Leuchtelement 104 ist im vorliegenden Fall als eine Weißlichtlampe, insbesondere eine Xenon-Lichtquelle, welche beispielsweise mit Filtern versehen ist, um die für Xenon bekannten Lichtbänder abzumindern und eine homogene Weißlichtverteilung zu erzeugen, ausgebildet. In einem Betriebszustand beleuchtet die Lichtquelle 98 das mittels der Bildgebungsvorrichtung 10 abzubildende Untersuchungsgebiet mit dem Beleuchtungslicht. Das Beleuchtungslicht weist ein Beleuchtungsspektrum 100 auf (vgl. Fig. 4). Bei dem Leuchtelement kann es sich aber beispielsweise auch um eine LED, OLED, Laser, insbesondere Diodenlaser, oder dergleichen handeln. Auch kann die Lichtquelle mehrere Leuchtelemente aufweisen, welche unterschiedliche Abstrahlcharakteristiken aufweisen können. Durch individuelle Ansteuerung bzw. Ein- und/oder Ausschalten solcher Leuchtelemente könnte ein Beleuchtungsspektrum variiert werden. Alternativ könnte es sich bei dem Leuchtelement auch um einen spektral durchstimmbares Leuchtelement handeln, wie beispielsweise einen durchstimmbaren Laser.

Die Bildgebungsvorrichtung 10 weist wenigstens einen Lichtwellenleiter 116 auf (vgl. Fig. 2). Im vorliegenden Fall weist die Bildgebungsvorrichtung 10 mehrere Lichtwellenleiter 116 auf. Der Übersichtlichkeit halber ist in den Zeichnungen nur ein Lichtwellenleiter 116 mit einem Bezugszeichen versehen und in der Beschreibung näher beschrieben. Der Lichtwellenleiter 116 ist dazu eingerichtet, das von der Lichtquelle 98 bereitgestellte Beleuchtungslicht entlang dem Schaft 16 von einem proximalen Ende zu einem distalen Ende zu leiten, um das Untersuchungsgebiets zu beleuchten. Der Lichtwellenleiter 116 ist in dem Überschaft 106 angeordnet. Der Lichtwellenleiter 116 ist in einem Zwischenbereich zwischen dem Schaft 16 und dem Überschaft 106 angeordnet. Der Lichtwellenleiter 116 weist im vorliegenden Fall mehrere Lichtleitfasern (nicht dargestellt) auf. Einzelne Lichtleitfasern könnten dabei individuell mit einzelnen Leuchtelementen der Lichtquelle verbunden sein. Alternativ könnten ein oder mehrere Leuchtelemente der Lichtquelle am distalen Endabschnitt des Überschaft angeordnet sein, wodurch auf die Lichtwellenleiter verzichtet werden könnte.

Der Bildergebungskanal 18 weist zur Erfassung wenigstens einer der Teilabbildungen 26, 30, 42, 46, 62, 66 der Bildgebungszweige 20, 38, 58 wenigstens eine Kamera 96 auf. Die Kamera 96 ist in einem proximalen und zwar insbesondere extrakorporalen Endabschnitts 121 des Schafts 16 der Bildgebungsvorrichtung 10 angeordnet. Die Kamera 96 weist ein Kameragehäuse 168 auf. In dem Kameragehäuse 168 sind weitere Komponenten der Kamera 96 angeordnet. Im vorliegenden Fall ist die Kamera 96 als eine hyperspektrale Kamera ausgebildet. Alternativ könnte die Kamera jedoch als Multispektralkamera oder Weißlichtkamera ausgebildet sein.

Fig. 3 zeigt einen Aufbau der Kamera 96 in einer schematischen Darstellung. Die Kamera 96 weist wenigstens ein Eingangsobjektiv 170 auf. Das Eingangsobjektiv 170 ist in dem Kameragehäuse 168 angeordnet. Das Eingangsobjektiv 170 ist den Bildgebungszweigen 20, 38 ,58 lichtstromaufwärts nachgeschaltet. Das Eingangsobjektiv 170 ist dem dritten Bildgebungszweig 58 nachgeschaltet. Das Eingangsobjektiv 170 sitzt hinter der dritten Relaisoptik 72. Das Eingangsobjektiv 170 ist dazu eingerichtet, eine Teilabbildung des zu untersuchenden Untersuchungsgebiets in einer Bildebene zur Erzeugung.

Die Kamera 96 weist eine Spektrometer 172 auf. Das Spektrometer 172 ist zu einer Ansteuerung mit dem Steuergerät 102 verbunden. Das Spektrometer 172 ist in dem Kameragehäuse 168 angeordnet. Das Spektrometer 172 ist lichtstromaufwärts hinter dem Eingangsobjektiv 170 angeordnet.

Das Spektrometer 172 weist wenigstens eine Blende 174 auf. Das Eingangsobjektiv 170 fokussiert 170 die Abbildung auf die Blende 174. Die Blende 174 ist in einer Bildebene des von dem Eingangsobjektiv 170 erzeugten Bildes angeordnet. Ein Abstand des Eingangsobjektivs 170 und der Blende 174 entspricht zumindest im Wesentlichen der Bildweite des Eingangsobjektivs 170. Die Blende 174 liegt in der Bildebene. Die Blende 174 ist dazu eingerichtet, einen Bereich des von dem Eingangsobjektiv 170 erzeugten Bildes zu selektieren. Dazu weist die Blende 174 eine Öffnung auf. Die Öffnung weist die Form eines Schlitzes auf. Eine Haupterstreckungsrichtung der Öffnung definiert eine erste Richtung. Diese erste Richtung ist zumindest im Wesentlichen parallel zur Bildebene des von dem Eingangsobjektiv 170 erzeugten Bildes. Die Blende 174 ist dazu eingerichtet, einen Streifen des Bildes zu selektieren, welcher einer Breite von wenigstens 15 µm und/oder von höchstens 30 µm aufweist.

Das Spektrometer 172 weist eine interne Optik 176 auf. Die interne Optik 176 ist lichtstromaufwärts hinter der Blende 174 angeordnet. Die interne Optik 176 weist wenigstens eine interne Linse 178 auf. Diese interne Linse 178 ist lichtstromaufwärts hinter der Blende 174 angeordnet. Ein Abstand der internen Linse 178 zu der Blende 174 entspricht der Brennweite der internen Linse 178. Derart bildet die interne Linse 178 die Blende 174 ins Unendliche ab.

Ferner weist das Spektrometer 172 wenigstens ein dispersives Element 180 auf. Das dispersive Element 180 ist lichtstromaufwärts hinter der internen Linse 178 angeordnet. Das dispersive Element 180 ist zu einer wellenlängenabhängigen Auffächerung von Licht vorgesehen. Im vorliegenden Fall ist das dispersive Element 180 dazu eingerichtet, dass dieses Licht in einer zweiten Richtung aufzufächern ist. Die zweite Richtung ist zumindest im Wesentlichen senkrecht zu der Haupterstreckung der Öffnung der Blende 174. Beispielsweise kann es sich bei dem dispersiven Element 180 um ein Prisma handeln. Im vorliegenden Fall ist das dispersive Element 180 als ein optisches Gitter, insbesondere Blaze-Gitter ausgebildet.

Die interne Optik 176 weist wenigstens eine weitere interne Linse 182 auf. Die weitere Objektivlinse 182 ist lichtstromaufwärts hinter dem dispersiven Element 180 angeordnet. Derart ist das dispersive Element 180 zwischen der internen Linse 178 und der weiteren internen Linse 182 angeordnet. In anderen Worten ist das dispersive Element 180 innerhalb der internen Optik176 angeordnet. Ein Abstand der weiteren internen Linse 182 zu dem dispersiven Element 180 entspricht der Brennweite der weiteren internen Linse 182. Die weitere interne Linse 182 ist dazu eingerichtet, das von dem dispersive Element 180 aufgefächerte Licht scharf abzubilden.

Das Spektrometer 172 weist einen Kamerabildsensor 184 auf. Der Kamerabildsensor 184 ist mit der Steuereinrichtung 102 verbunden. Der Kamerabildsensor 184 ist lichtstromaufwärts hinter der weiteren internen Linse 182 angeordnet. In anderen Worten ist die weitere interne Linse 182 zwischen dem dispersiven Element 180 und dem Kamerabildsensor 184 angeordnet. Der Kamerabildsensor 184 ist ein monochromer Sensor. Ein solcher monochromer Sensor weist lediglich eine einzige Spektralempfindlichkeit auf. Bei dem Kamerabildsensor 184 handelt es sich um einen zweidimensionalen CMOS-Kamerabildsensor. Alternativ könnte es sich um einen CCD-Kamerabildsensor handeln.

Die Kamera 96 weist eine Verstelleinrichtung 186 auf. Die Verstelleinrichtung 186 ist zu einer Steuerung mit der Steuereinrichtung 102 verbunden. Die Verstelleinrichtung 186 ist in dem Kameragehäuse 168 angeordnet. Die Verstelleinrichtung 186 ist dazu eingerichtet, wenigstens die Blende 174 relativ zu dem Eingangsobjektiv 170 zu verstellen. Im vorliegenden Fall wird das gesamte Spektrometer 172 relativ zu dem Eingangsobjektiv 170 verstellt. Die Verstelleinrichtung 186 weist wenigstens ein Lager auf. Das Lager ist zu einer beweglichen Lagerung des Spektrometers 172 relativ zu dem Eingangsobjektiv 170 eingerichtet. Im vorliegenden Fall ist das Lager als ein Linearlager ausgebildet. Beispielsweise kann das Lager Führungsschienen umfassen, welche entlang der zweiten Richtung erstreckend angeordnet sind. Die Verstelleinrichtung 186 weist ferner einen Verstellaktor zum Antrieb auf. Der Verstellaktor ist im vorliegenden Fall als ein Linearaktor ausgebildet. Um beispielsweise ein gleichmäßiges Verstellen erzielen zu können, muss der Verstellaktor als ein Piezoaktor ausgebildet sein.

Durch das Verstellen der Blende 174 relativ zu dem Eingangsobjektiv 170 können für verschiedene Bildausschnitte des zu untersuchenden Untersuchungsgebiets Spektren aufgezeichnet werden. Durch verschieben kann somit das gesamte Untersuchungsgebiet spektral abgetastet werden, wodurch sich ein Abbild inklusive spektraler Informationen erzeugen lässt.

Die Kamera 96 ermöglicht eine spektral höher aufgelöste Darstellung des Untersuchungsgebiets. Allerdings kann eine Bildrate durch diese Technik relativ zu anderen Methoden niedrig sein. Die Kamera 96 wird in einem Verfahren zur Feindiagnostik eingesetzt. Beispielsweise dann, falls in einem mittels Bildsensoren der Bildgebungszweige des Bildgebungskanals aufgenommenen Farbbildern verifiziert werden müssen.

Fig. 4 zeigt ein schematisches Diagramm, welches die spektralen Eigenschaften der Lichtquelle 98 darstellt. Das Diagramm zeigt eine Abszissenachse. Auf der Abszissenachse ist eine Wellenlänge aufgetragen. Ferner zeigt das Diagramm eine Ordinatenachse. Auf der Ordinatenachse ist eine Intensität aufgetragen. Das Diagramm zeigt ferner eine Beleuchtungskennlinie. Die Beleuchtungskennlinie 140 charakterisiert das Beleuchtungsspektrum 100 der Lichtquelle 98. Die Beleuchtungskennlinie 140 zeigt im vorliegenden Fall einen für Weißlicht charakteristischen Verlauf.

Fig. 5 zeigt ein schematisches Diagramm, welches beispielhafte spektrale Eigenschaften des Untersuchungsgebiets darstellt. Das Diagramm zeigt eine Abszissenachse. Auf der Abszissenachse ist eine Wellenlänge aufgetragen. Ferner zeigt das Diagramm eine Ordinatenachse. Auf der Ordinatenachse ist eine Intensität aufgetragen.

Das Diagramm zeigt eine erste Spektralkennlinie 142. Die erste Spektralkennlinie 142 charakterisiert die spektralen Eigenschaften des Untersuchungsgebiet. Die erste Spektralkennlinie 142 zeigt im vorliegenden Fall einen für Fettgewebe charakteristischen Verlauf auf.

Das Diagramm zeigt ferner eine zweite Spektralkennlinie 144. Die zweite Spektralkennlinie 144 charakterisiert die spektralen Eigenschaften des Untersuchungsgebiets. Die zweite Spektralkennlinie 144 zeigt im vorliegenden Fall einen für ein wässriges Gewebe charakteristischen Verlauf auf.

Das Diagramm zeigt ferner eine dritte Spektralkennlinie 146. Die dritte Spektralkennlinie 146 charakterisiert die spektralen Eigenschaften des Untersuchungsgebiets. Die dritte Spektralkennlinie 146 zeigt im vorliegenden Fall einen für deoxygeniertes Gewebe charakteristischen Verlauf auf.

Das Diagramm zeigt ferner eine vierte Spektralkennlinie 148. Die vierte Spektralkennlinie 148 charakterisiert die spektralen Eigenschaften des Untersuchungsgebiet. Die vierte Spektralkennlinie 148 zeigt im vorliegenden einen für oxygeniertes Gewebe charakteristischen Verlauf auf.

Über das gesamte Untersuchungsgebiet variieren die spektralen Eigenschaften des Gewebes. Somit kann anhand dieser auf eine Gewebeart und/oder eine Gewebeeigenschaft des Untersuchungsgebiets rückgeschlossen werden. Im vorliegenden Fall handelt es sich bei den Spektrallinien 142, 144, 146, 148 um Reflexionsspektren des Untersuchungsgebiets. Alternativ könnten aber auch Transmissionsspektren, Fluoreszenz- und/oder Phosphoreszenzspektren genutzt werden. Dies kann von einem Betriebsmodus abhängig sein. Je nach Betriebsmodus könnte als Antwort des Untersuchungsgebiets auf das eingestrahlte Beleuchtungslicht eine Reflexion, Transmission, Fluoreszenz und/oder Phosphoreszenz untersucht werden.

Fig. 6 zeigt ein schematisches Diagramm, in welchem optische Eigenschaften der Strahlteiler 22, 40, 60 der Bildgebungszweige 20, 38, 58 des Bildgebungskanals 18 idealisiert dargestellt sind (vgl. Fig. 2). Das Diagramm zeigt eine Abszissenachse. Auf der Abszissenachse ist eine Wellenlänge aufgetragen. Ferner zeigt das Diagramm eine Ordinatenachse. Auf der Ordinatenachse ist eine Intensität aufgetragen. Auf der Ordinatenachse ist eine Transmissionsintensität aufgetragen. Die Transmissionsintensität beschreibt den Anteil des auf den Strahlteiler 22, 40, 60 fallenden Lichts, welcher von den Strahlteilern 22, 40, 60 transmittiert wird.

Das Diagramm zeigt eine erste Transmissionskennlinie 150. Die erste Transmissionskennlinie 150 beschreibt das spektrale Transmissionsverhalten des ersten spektralselektiven Strahlteilers 22. Die erste Transmissionskennlinie 150 charakterisiert die spektrale Aufteilung der Abbildung des Untersuchungsgebiets in die erste spektrale Teilabbildung 26 und die weitere erste spektrale Teilabbildung 30 durch den ersten spektralselektiven Strahlteiler 22. Die erste Transmissionskennlinie 150 charakterisiert die spektrale Aufteilung einer ursprünglichen spektralen Antwort des Untersuchungsgebiets auf das Beleuchtungslicht in den ersten Spektralbereich 28 und den weiteren ersten Spektralbereich 32 durch den ersten spektralselektiven Strahlteiler 22. Im ersten Spektralbereich 28 beträgt der Transmissionsanteil höchstens 25%. Im vorliegenden Fall sogar höchstens 5%. Im weiteren ersten Spektralbereich 32 beträgt der Transmissionsanteil wenigstens 75%. Im vorliegenden Fall sogar wenigstens 95%. Der erste Spektralbereich 28 und der weitere erste Spektralbereich 32 sind durch eine Flanke der Transmissionskennlinie voneinander getrennt. Die Flanke steigt vom ersten Spektralbereich 28 bis zum weiteren ersten Spektralbereich 32 an. Die Flanke weist einen Flankenwendepunkt auf. Der Flankenwendepunkt liegt bei zumindest im Wesentlichen 540 nm. Es ist jedoch auch denkbar, dass die Spektralbereiche miteinander vertauscht sein könnten. Auch könnte die Transmissionskennlinie mehrere Spektralbereiche definieren, welche zueinander spektral versetzt sein könnten.

Das Diagramm zeigt eine zweite Transmissionskennlinie 152. Die zweite Transmissionskennlinie 152 beschreibt das spektrale Transmissionsverhalten des zweiten spektralselektiven Strahlteilers 40. Die zweite Transmissionskennlinie 152 charakterisiert die spektrale Aufteilung der weiteren ersten Teilabbildung 30 in die zweite spektrale Teilabbildung 42 und die weitere zweite spektrale Teilabbildung 46 durch den zweiten spektralselektiven Strahlteiler 40. Die zweite Transmissionskennlinie 152 charakterisiert die spektrale Aufteilung des weiteren ersten Spektralbereichs 32 in den zweiten Spektralbereich 44 und den weiteren zweiten Spektralbereich 48 durch den zweiten spektralselektiven Strahlteiler 40. Im zweiten Spektralbereich 44 beträgt der Transmissionsanteil höchstens 25%. Im vorliegenden Fall sogar höchstens 5%. Im weiteren zweiten Spektralbereich 48 beträgt der Transmissionsanteil wenigstens 75%. Im vorliegenden Fall sogar wenigstens 95%. Der zweite Spektralbereich 44 und der weitere zweite Spektralbereich 48 sind durch eine Flanke der Transmissionskennlinie 152 voneinander getrennt. Die Flanke steigt vom zweiten Spektralbereich 44 bis zum weiteren zweiten Spektralbereich 48 an. Die Flanke weist einen Flankenwendepunkt auf. Der Flankenwendepunkt liegt bei zumindest im Wesentlichen 680 nm. Es ist jedoch auch denkbar, dass die Spektralbereiche miteinander vertauscht sein könnten. Auch könnte die Transmissionskennlinie mehrere Spektralbereiche definieren, welche zueinander spektral versetzt sein könnten.

Das Diagramm zeigt eine dritte Transmissionskennlinie 154. Die dritte Transmissionskennlinie 154 beschreibt das spektrale Transmissionsverhalten des dritten spektralselektiven Strahlteilers 60. Die dritte Transmissionskennlinie 154 charakterisiert die spektrale Aufteilung der weiteren zweiten Teilabbildung 46 in die dritte spektrale Teilabbildung 62 und die weitere dritte spektrale Teilabbildung 66 durch den dritten spektralselektiven Strahlteiler 60. Die dritte Transmissionskennlinie 154 charakterisiert die spektrale Aufteilung des weiteren zweiten Spektralbereichs 48 in den dritten Spektralbereich 64 und den weiteren dritten Spektralbereich 68 durch den dritten spektralselektiven Strahlteiler 60.Im dritten Spektralbereich 64 beträgt der Transmissionsanteil höchstens 25%. Im vorliegenden Fall sogar höchstens 5%. Im weiteren dritten Spektralbereich 68 beträgt der Transmissionsanteil wenigstens 75%. Im vorliegenden Fall sogar wenigstens 95%. Der dritte Spektralbereich 64 und der weitere dritte Spektralbereich 68 sind durch eine Flanke der dritten Transmissionskennlinie 154 voneinander getrennt. Die Flanke steigt vom dritten Spektralbereich 64 bis zum weiteren dritten Spektralbereich 68 an. Die Flanke weist einen Flankenwendepunkt auf. Der Flankenwendepunkt liegt bei zumindest im Wesentlichen 670 nm. Es ist jedoch auch denkbar, dass die Spektralbereiche miteinander vertauscht sein könnten. Auch könnte die Transmissionskennlinie mehrere Spektralbereiche definieren, welche zueinander spektral versetzt sein könnten.

Fig. 7 zeigt ein schematisches Diagramm, in welchem weitere optische Eigenschaften der Strahlteiler 22, 40, 60 der Bildgebungszweige 20, 38, 58 des Bildgebungskanals 18 idealisiert dargestellt sind. Das Diagramm zeigt eine Abszissenachse. Auf der Abszissenachse ist eine Wellenlänge aufgetragen. Ferner zeigt das Diagramm eine Ordinatenachse. Auf der Ordinatenachse ist eine Intensität aufgetragen. Im Falle von Fig. 7 ist auf der Ordinatenachse eine Reflexionsintensität aufgetragen. Der Reflexionsanteil beschreibt den Anteil von auf den Strahlteiler 22, 40, 60 einfallenden Lichts, welcher von dem Strahlteiler 22, 40, 60 reflektiert wird. Das Reflexionsverhalten der Strahlteiler 22, 40, 60 ist bis auf einen Absorptionsanteil, welcher in etwa vernachlässigbar ist, im Wesentlichen komplementär zu dem oben beschriebenen Transmissionsverhalten.

Das Diagramm zeigt eine erste Reflexionskennlinie 156. Die erste Reflexionskennlinie 156 beschreibt das spektrale Reflexionsverhalten des ersten spektralselektiven Strahlteilers 22. Die erste Reflexionskennlinie 156 charakterisiert die spektrale Aufteilung der Abbildung des Untersuchungsgebiets in die erste spektrale Teilabbildung 26 und die weitere erste spektrale Teilabbildung 30 durch den ersten spektralselektiven Strahlteiler 22. Die erste Reflexionskennlinie 156 charakterisiert die spektrale Aufteilung einer ursprünglichen spektralen Antwort des Untersuchungsgebiets auf das Beleuchtungslicht in den ersten Spektralbereich 28 und den weiteren ersten Spektralbereich 32 durch den ersten spektralselektiven Strahlteiler 22. Im ersten Spektralbereich 28 beträgt der Reflexionsanteil wenigstens 75%. Im vorliegenden Fall sogar wenigstens 95%. Im weiteren ersten Spektralbereich 32 beträgt der Reflexionsanteil höchstens 25%. Im vorliegenden voll sogar höchstens 5%. Der erste Spektralbereich 28 und der weitere erste Spektralbereich 32 sind durch eine Flanke der Reflexionskennlinie 156 voneinander getrennt. Die Flanke fällt vom ersten Spektralbereich 28 bis zum weiteren ersten Spektralbereich 32 ab. Die Flanke weist einen Flankenwendepunkt auf. Der Flankenwendepunkt liegt bei zumindest im Wesentlichen 540 nm.

Es ist jedoch auch denkbar, dass die Spektralbereiche miteinander vertauscht sein könnten. Auch könnte die Transmissionskennlinie mehrere Spektralbereiche definieren, welche zueinander spektral versetzt sein könnten.

Das Diagramm zeigt eine zweite Reflexionskennlinie 158. Die zweite Reflexionskennlinie 158 beschreibt das spektrale Reflexionsverhalten des zweiten spektralselektiven Strahlteilers 40. Die zweite Reflexionskennlinie 158 charakterisiert die spektrale Aufteilung der weiteren ersten Teilabbildung 30 in die zweite spektrale Teilabbildung 42 und die weitere zweite spektrale Teilabbildung 46 durch den zweiten spektralselektiven Strahlteiler 40. Die zweite Reflexionskennlinie 158 charakterisiert die spektrale Aufteilung des weiteren ersten Spektralbereichs 32 in den zweiten Spektralbereich 44 und den weiteren zweiten Spektralbereich 48 durch den zweiten spektralselektiven Strahlteiler 40. Im zweiten Spektralbereich 44 beträgt der Reflexionsanteil wenigstens 75%. Im vorliegenden Fall sogar wenigstens 95%. Im weiteren zweiten Spektralbereich 48 beträgt der Reflexionsanteil höchstens 25%. Im vorliegenden Fall sogar höchstens 5%. Der zweite Spektralbereich 44 und der weitere zweite Spektralbereich 48 sind durch eine Flanke der zweiten Reflexionskennlinie 158 voneinander getrennt. Die Flanke fällt vom zweiten Spektralbereich 44 bis zum weiteren zweiten Spektralbereich 48 an. Die Flanke weist einen Flankenwendepunkt auf. Der Flankenwendepunkt liegt bei zumindest im Wesentlichen 680 nm. Es ist jedoch auch denkbar, dass die Spektralbereiche miteinander vertauscht sein könnten. Auch könnte die Transmissionskennlinie mehrere Spektralbereiche definieren, welche zueinander spektral versetzt sein könnten.

Das Diagramm zeigt eine dritte Reflexionskennlinie 160 auf. Die dritte Reflexionskennlinie 160 beschreibt das spektrale Reflexionsverhalten des dritten spektralselektiven Strahlteilers 60. Die dritte Reflexionskennlinie 160 charakterisiert die spektrale Aufteilung der weiteren zweiten Teilabbildung 46 in die dritte spektrale Teilabbildung 62 und die weitere dritte spektrale Teilabbildung 66 durch den dritten spektralselektiven Strahlteiler 60. Die dritte Reflexionskennlinie 160 charakterisiert die spektrale Aufteilung des weiteren zweiten Spektralbereichs 48 in den dritten Spektralbereich 64 und den weiteren dritten Spektralbereich 68 durch den dritten spektralselektiven Strahlteiler 60. Im dritten Spektralbereich 64 beträgt der Reflexionsanteil wenigstens 75%. Im vorliegenden Fall sogar wenigstens 95%. Im weiteren dritten Spektralbereich 68 beträgt der Reflexionsanteil höchstens 25%. Im vorliegenden Fall sogar höchstens 5%. Der dritte Spektralbereich 64 und der weitere dritte Spektralbereich 68 sind durch eine Flanke der dritten Transmissionskennlinie 154 voneinander getrennt. Die Flanke fällt vom dritten Spektralbereich 64 bis zum weiteren dritten Spektralbereich 68 ab. Die Flanke weist einen Flankenwendepunkt auf. Der Flankenwendepunkt liegt bei zumindest im Wesentlichen 670 nm. Es ist jedoch auch denkbar, dass die Spektralbereiche miteinander vertauscht sein könnten. Auch könnte die Transmissionskennlinie mehrere Spektralbereiche definieren, welche zueinander spektral versetzt sein könnten.

Derart unterteilen die Strahlteiler 20, 44, 60 Licht der Abbildung in jeweilige Teilabbildungen 22, 30, 42, 46, 62, 66 und ihre jeweiligen Teilspektralbereiche.

In Fig. 8 sind Detektionseigenschaften der Bildsensoren 34, 50, 70 anhand eines Diagramms dargestellt. Im vorliegenden Fall sind die Bildsensoren 34, 50, 70 im Wesentlichen identisch zueinander ausgebildet. Alternativ könnten die Bildsensoren wenigstens teilweise verschieden voneinander ausgebildet sein und beispielsweise über unterschiedliche Detektionseigenschaften verfügen.

Das Diagramm in Fig. 8 zeigt eine Abszissenachse. Auf der Abszissenachse ist eine Wellenlänge aufgetragen. Ferner zeigt das Diagramm eine Ordinatenachse. Auf der Ordinatenachse ist eine Intensität aufgetragen. Im Falle von Fig. 8 ist auf der Ordinatenachse eine Signalintensität aufgetragen. Diese beschreibt mit welcher Signalstärke ein Sensor Licht einer gewissen Wellenlänge sensiert. Im Folgenden sind die Eigenschaften des Bildsensors 34, 50, 70 anhand eines der Bildsensoren 34, 50, 70 näher beschrieben. Diese Beschreibung ist auf die anderen Bildsensoren 34, 50, 70 übertragbar.

Der erste Bildsensor 34 weist wenigstens eine erste Spektralempfindlichkeit 88 auf. Das Diagramm zeigt eine erste Empfindlichkeitskennlinie 162, welche die erste Spektralempfindlichkeit 88 charakterisiert. Die erste Spektralempfindlichkeit 88 entspricht einem blauen Spektralband eines RGB-Sensors.

Ferner weist der erste Bildsensor 34 wenigstens eine zweite Spektralempfindlichkeit 90 auf. Das Diagramm zeigt eine zweite Empfindlichkeitskennlinie 164, welche die zweite Spektralempfindlichkeit 90 charakterisiert. Die zweite Spektralempfindlichkeit 90 entspricht einem grünen Spektralband eines RGB-Sensors.

Ferner weist der erste Bildsensor 34 wenigstens eine dritte Spektralempfindlichkeit 92 auf. Das Diagramm zeigt eine dritte Empfindlichkeitskennlinie 166, welche die dritte Spektralempfindlichkeit 92 charakterisiert. Die dritte Spektralempfindlichkeit 92 entspricht einem roten Spektralband eines RGB-Sensors.

Alternativ könnten die Spektralempfindlichkeiten auch ein anderes spektrales Verhalten zeigen. Beispielsweise könnten dies Ultraviolette, Nahinfrarote, Infrarote Spektralbänder eines Sensors darstellen.

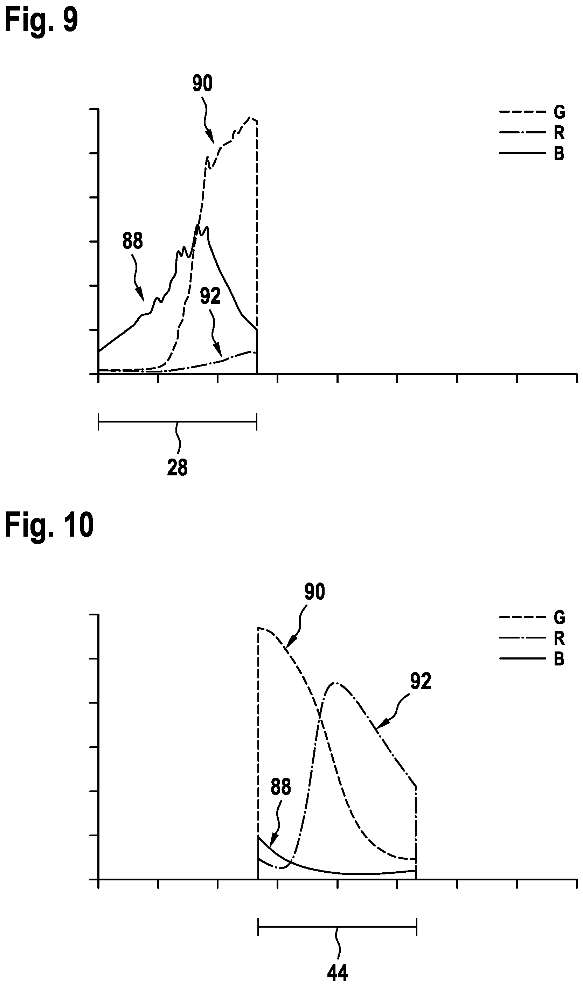

In den Fig. 9, 10,11 sind die Spektralempfindlichkeiten 88, 90, 92 der Bildsensoren 34, 50, 70 innerhalb der den jeweiligen Sensoren zugeordneten Teilspektralbereichen dargestellt. Durch Vergleich der Fig. 4 mit den Fig. 9, 10,11 ist ersichtlich, dass das Beleuchtungsspektrum 100 einen Spektralbereich umfasst, welcher breiter ist, als wenigstens ein Spektralbereich der Teilabbildungen 22, 30, 42, 46, 62, 66 der Bildgebungszweige 20, 38, 58. Im vorliegenden Fall ist das Beleuchtungsspektrum 100 breiter als der erste Spektralbereich 28, der zweite Spektralbereich 44 und der dritte Spektralbereich 64. Bei einer Messung ergibt sich aus der Überlagerung der spektralen Antwort des Untersuchungsgebiets mit den Spektralbereichen 28, 44, 64 innerhalb der jeweiligen Teilspektralbereich jeweils drei Stützstellen, welche zu einer Analyse der Art und/oder Eigenschaft des Untersuchungsgebiets genutzt werden.

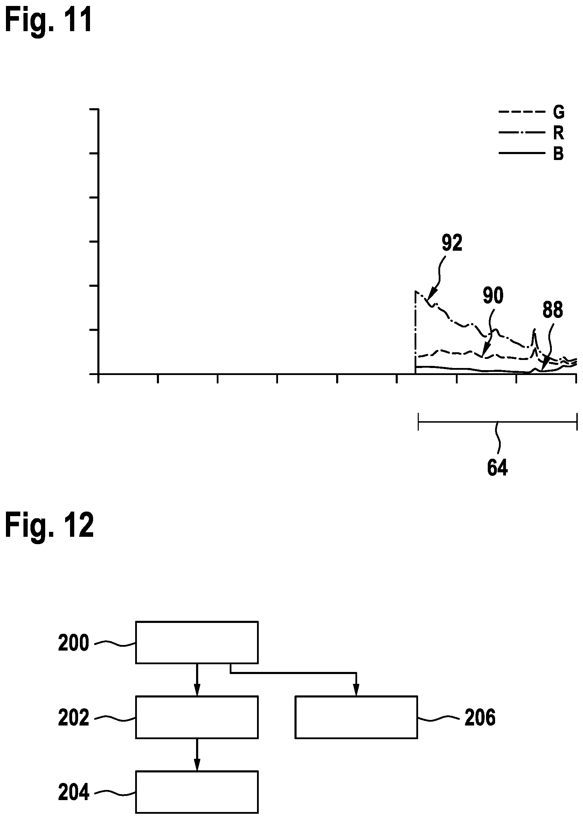

Fig. 12 zeigt einen schematischen Ablaufplan eines beispielhaften, aber nicht beanspruchten Verfahrens zum Betrieb der endoskopischen Bildgebungsvorrichtung 10. Dieses kann unmittelbar auch auf einen Betrieb einer exoskopischen Vorrichtung übertragen werden.

Das Verfahren umfasst wenigstens einen Verfahrensschritt 200. In dem Verfahrensschritt 200 wird die endoskopische Vorrichtung auf ein Untersuchungsgebiet ausgerichtet. Die Lichtquelle 98 wir aktiviert. Das Licht trifft auf das Untersuchungsgebiet. Das Licht wird wenigstens teilweise von dem Untersuchungsgebiet reflektiert. Ferner wird das Licht wenigstens teilweise von dem Untersuchungsgebiet absorbiert und/oder transmittiert. Ferner kann das Untersuchungsgebiet als Antwort auf das Licht Fluoreszenz oder Phosphoreszenz abstrahlen. Das von dem Untersuchungsgebiet reflektierte bzw. abgestrahlte Licht wird mittels der endoskopischen Vorrichtung eingefangen. Dabei gelang das Licht in den Bildgebungskanal 18. In dem Bildungskanal wir eine Abbildung des Untersuchungsgebiets erzeugt. Diese Abbildung wird durch die Bildgebungszweige 20, 38, 58 in die Teilabbildungen 22, 30, 42, 46, 62, 66 jeweiliger Teilspektralbereiche aufgeteilt. Die Teilabbildungen 22, 30, 42, 46, 62, 66 jeweiliger Teilspektralbereiche werden von den jeweiligen zugeordneten Bildsensoren 34, 50, 70 erfasst. Die Bildsensoren 34, 50, 70 geben entsprechend ihrer Spektralempfindlichkeiten 88, 90, 92 Stützstellen aus. Die Signale der Bildsensoren 34, 50, 70 werden an die Steuereinrichtung übermittelt.

Das Verfahren umfasst einen weiteren Verfahrensschritt 202. In dem weiteren Verfahrensschritt 202 vergleicht die Steuereinrichtung 120 die Stützstellen mit in dem Speicher hinterlegten Vergleichswerten, beispielsweise mit für verschiedene Gewebearten und/oder Eigenschaften charakteristischen Kurvenverläufen, wie in Fig. 5 dargestellt. Auf Basis der Stützstellen ordnet die Steuereinrichtung 102 diese Stützstellen dann den verschiedenen Gewebearten und/oder Eigenschaften zu. Dies wird für jeden Pixel bzw. jede RGB-Pixelgruppe eines jeweiligen Bildsensors 34, 50, 70 durchgeführt.

Das Verfahren umfasst einen weiteren Verfahrensschritt 204. In dem Verfahrensschritt 204 hinterlegt die Steuereinrichtung 102 die Zuordnung in einem Speicher oder gibt dies mittels eines farbcodierten Bildes aus. Dabei kann für jede Zuordnung der Gewebeeigenschaft und/oder Art ein einzelnes Bild ausgegeben werden oder in einem gemeinsamen farbcodierten Bild. Die einzelnen Bilder können auf der Anzeige gleichzeitig, einzeln oder insbesondere alternierend dargestellt werden. Ferner findet die Aufnahme, Erzeugung und/oder Darstellung der Farbbilder mit einer Bildrate von wenigstens 15 Bildern pro Sekunde statt. Somit wird ein Video erzeugt, welches sich aus den Farbbildern zusammensetzt und vorteilhaft in Echtzeit das Untersuchungsgebiet darstellt.

Das Verfahren umfasst wenigstens einen weiteren Verfahrensschritt 206. In dem Verfahrensschritt 206 erzeugt die Steuereinrichtung 102 durch Überlagerung der Teilabbildungen 22, 30, 42, 46, 62, 66 ein Weißlichtbild des Untersuchungsgebiets. Dieses Untersuchungsgebiet kann separat zu dem einzelnen Bild bzw. farbcodierten Bild der Zuordnung der Gewebeeigenschaft und/oder Art angezeigt werden. Alternativ oder zusätzlich ist es denkbar dieses jedoch miteinander in einem Überlagerungsbild miteinander überlagert auszugeben. Ferner könnte das Weißlichtbild, einzelne Bilder und/oder das gemeinsame farbcodierte Bild alternierend angezeigt werden.

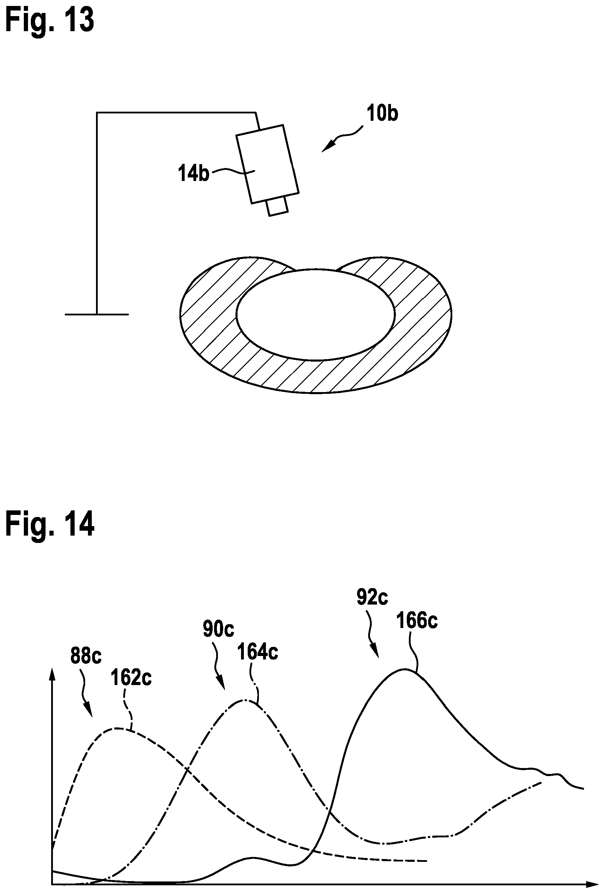

In den Figuren 13 bis 16 sind weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 12 verwiesen wird. Zur Unterscheidung der Ausführungsbeispiele ist der Buchstabe a den Bezugszeichen des Ausführungsbeispiels in den Figuren 1 bis 12 nachgestellt. In den Ausführungsbeispielen der Figuren 13 bis 18 ist einem jeweiligen Bezugszeichen zur Unterscheidung zu dem vorhergehenden Ausführungsbeispiel der Buchstaben b, c, d, e, f und g hintenangestellt.

Fig. 13 zeigt eine schematische Darstellung einer exoskopischen Bildgebungsvorrichtung 10b in einer perspektivischen Ansicht. Die vorliegende Ausgestaltung unterscheidet ich von der vorhergehenden im Wesentlichen durch eine Ausgestaltung des Schafts 16b. Dieser ist im Falle einer exoskopischen Vorrichtung nicht zu einem Einführen in eine Kavität eingerichtet. Die exoskopische Bildgebungsvorrichtung 10b bildet ein Exoskop 14b vollständig aus. Alternativ könnte die exoskopische Bildgebungsvorrichtung nur einen funktionsfähigen Bestandteil eines Exoskops ausbilden. Mittels einer solchen exoskopischen Ausgestaltung können auch von außerhalb einer Kavität multispektrale und/oder hyperspektrale Bilder in einem Untersuchungsgebiet der Kavität aufgenommen werden.

Fig. 14 zeigt optische Eigenschaften einer alternativen Bildgebungsvorrichtung 10c. Die vorliegende Bildgebungsvorrichtung 10c unterscheidet sich von den vorhergehenden im Wesentlichen durch voneinander verschieden ausgebildete Bildsensoren 34c, 50c, 70c.

Die Bildgebungsvorrichtung 10c weist im vorliegenden Fall wenigstens einen Bildgebungskanal 18c auf. Der Bildgebungskanal 18c umfasst wenigstens drei Bildgebungskanäle. Die Bildgebungskanäle weisen im vorliegenden Fall unterschiedliche Bildsensoren 34c, 50c, 70c auf. Die Bildsensoren 34c, 50c, 70c besitzen unterschiedliche Spektralempfindlichkeiten 88c, 90c, 92c. Fig. 12 zeigt ein Diagramm ähnlich dem der Fig. 7. Im vorliegenden Fall weisen die Bildsensoren 34c, 50c, 70c jedoch unterschiedliche Spektralempfindlichkeiten 88c, 90c, 92c auf. Sogar weist jeder Sensor jeweils nur eine Spektralempfindlichkeit 88c, 90c, 92c auf. Dadurch kann eine Überlagerung der Spektralempfindlichkeiten 88c, 90c, 92c mit Bezug auf verschiedene Teilspektralbereiche vermieden werden. Es ist jedoch auch denkbar, dass die Bildsensoren mehrere Spektralempfindlichkeiten 88c, 90c, 92c aufweisen, welche alle voneinander verschieden sind.

Der erste Bildsensor 34c weist wenigstens eine Spektralempfindlichkeit 88c auf. Das Diagramm zeigt eine erste Empfindlichkeitskennlinie 162c, welche die erste Spektralempfindlichkeit 88c charakterisiert. Die erste Spektralempfindlichkeit 88c entspricht einem blauen Spektralband eines RGB-Sensors.

Der zweite Bildsensor 50c weist wenigstens eine Spektralempfindlichkeit 90c auf. Das Diagramm zeigt eine zweite Empfindlichkeitskennlinie 164c, welche die zweite Spektralempfindlichkeit 90c charakterisiert. Die zweite Spektralempfindlichkeit 90c entspricht einem grünen Spektralband eines RGB-Sensors.

Der dritte Bildsensor 70c weist wenigstens eine Spektralempfindlichkeit 92c auf. Das Diagramm zeigt eine dritte Empfindlichkeitskennlinie 166c, welche die dritte Spektralempfindlichkeit 92c charakterisiert. Die dritte Spektralempfindlichkeit 92c: entspricht einem roten Spektralband eines RGB-Sensors.

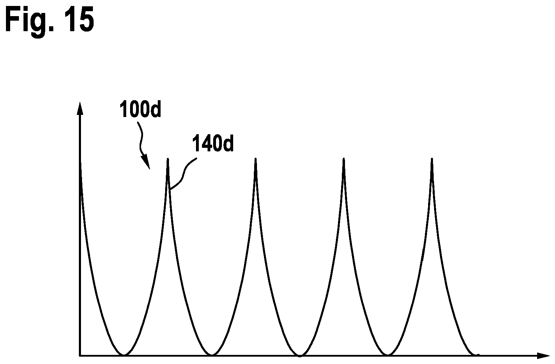

Fig. 15 zeigt optische Eigenschaften einer weiteren Ausgestaltung einer weiteren endoskopischen und/oder exoskopische Bildgebungsvorrichtung 10d. Das vorliegende Ausführungsbeispiel unterscheidet sich von der vorhergehenden im Wesentlichen durch eine Ausgestaltung der Lichtquelle 98d.

Die Lichtquelle 98d strahlt Beleuchtungslicht ab, welches wenigstens ein Beleuchtungsspektrum 100d umfasst, welches innerhalb wenigstens eines Spektralbereichs 28d, 32d, 44d, 48d, 64d, 68d von Teilabbildungen 22d, 30d, 42d, 46d, 62d, 66d von Bildgebungszweigen 20d, 38d, 58d eines Bildgebungskanals 18d der Bildgebungsvorrichtung 10d liegt (vgl. Fig. 5, 6). Das Beleuchtungsspektrum 100d weist eine Breite auf, welche geringer ist als der Spektralbereich 28d, 32d, 44d, 48d, 64d, 68d der Teilabbildung innerhalb welcher diese liegt. Die Lichtquelle kann beispielsweise als ein Lichtelement eine oder mehrere LEDs umfassen, welche allein, zusammen oder im Wechsel betrieben werden können.

Fig. 15 zeigt ein schematisches Diagramm, welches die spektralen Eigenschaften der Lichtquelle 98 darstellt. Das Diagramm zeigt eine Abszissenachse. Auf der Abszissenachse ist eine Wellenlänge aufgetragen. Ferner zeigt das Diagramm eine Ordinatenachse. Auf der Ordinatenachse ist eine Intensität aufgetragen. Das Diagramm zeigt ferner eine Beleuchtungskennlinie 140d der Lichtquelle 98d. Die Beleuchtungskennlinie 140d umfasst charakterisiert das Beleuchtungsspektrum 100d der Lichtquelle 98d. Die Beleuchtungskennlinie 140d zeigt im vorliegenden Fall einen für eine LED charakteristischen Verlauf. Die Beleuchtungskennlinie 140d hat verschiedene Hauptmaximas, welche beispielsweise zu einer spektralen Analyse von Fettgewebe, eines Wassergehalts, einer Oxygenierung einer Desoxygenierung oder dergleichen genutzt werden.

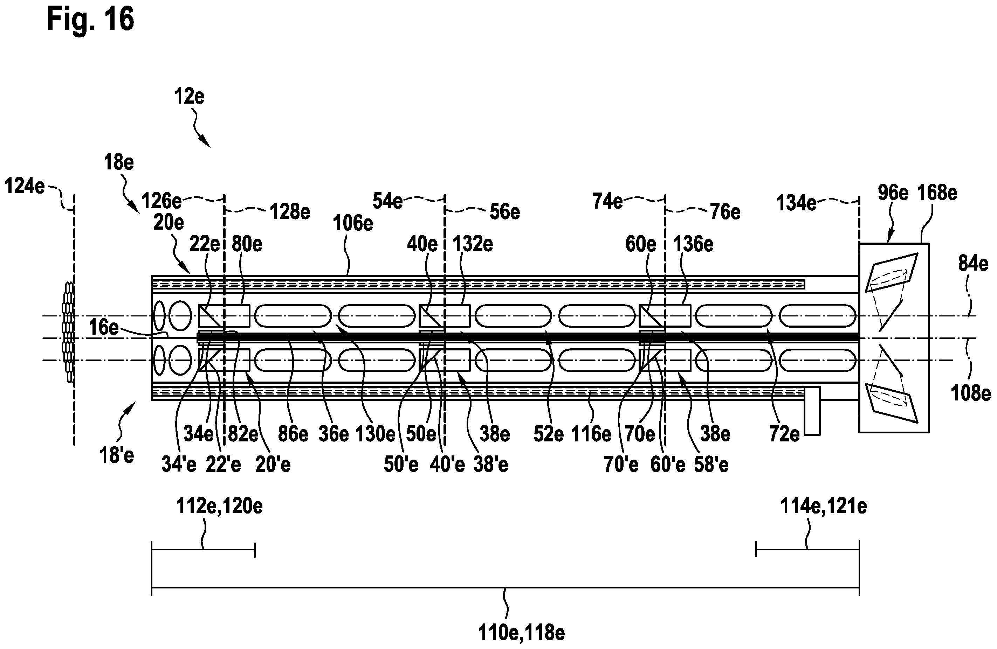

Fig. 16 zeigt eine schematische Darstellung einer alternativen endoskopischen und/oder exoskopischen Bildgebungsvorrichtung 10e in einer Schnittansicht. Die vorliegende Bildgebungsvorrichtung 10e unterscheidet sich von der vorhergehenden im Wesentlichen durch eine Anzahl von Bildgebungskanälen 18e, 18'e.

Im vorliegenden Fall weist die Bildgebungsvorrichtung 10e wenigstens einen Bildgebungskanal 18e auf. Ferner weist die Bildgebungsvorrichtung 10e wenigstens einen weiteren Bildgebungskanal 18'e auf. Insgesamt weist die Bildgebungsvorrichtung 10e zwei Bildgebungskanäle 18e, 18'e auf. Im Folgenden werden für die Komponenten der Bildgebungskanäle gleiche Bezugszeichen verwendet. Zur Unterscheidung sind den Bezugszeichen der Komponenten des weiteren Bildgebungskanal 18'e ein Apostroph nachgestellt. Die Verwendung zweier Bildgebungskanäle 18e, 18'e ermöglicht eine vorteilhafte stereoskopische multi- und/oder hyperspektrale Bildgebung. Auch kann durch die Verwendung mehrerer Bildgebungskanäle eine spektrale Auflösung weiter erhöht werden. Um eine räumliche Darstellung und/oder spektrale Auflösung weiter zu verbessern und/oder zu erhöhen ist es denkbar, dass die Bildgebungsvorrichtung über zusätzliche Bildgebungskanäle verfügen kann.

Der Bildgebungskanal 18e und der weitere Bildgebungskanal 18'e sind versetzt zueinander angeordnet. Die Bildgebungskanäle 18e, 18'e sind seitlich zueinander versetzt angeordnet. Die Bildgebungskanäle 18e, 18'e sind im Wesentlichen senkrecht zu Mittelachsen der jeweiligen Aufnahmeschäfte der Bildgebungskanäle 18e, 18'e versetzt zueinander angeordnet. Die Mittelachsen der Bildgebungskanäle 18e, 18'e sind zumindest im Wesentlichen parallel zueinander. Blickrichtungen der Bildgebungskanäle 18e, 18'e sind zumindest im Wesentlichen parallel. Alternativ könnten die Mittelachsen der Bildgebungskanäle jedoch auch winklig zueinander angeordnet sein. Blickrichtungen der Bildgebungskanäle könnten auch winklig zueinander sein.

Der Bildgebungskanal 18e und der weitere Bildgebungskanal 18'e sind zueinander spiegelsymmetrisch angeordnet. Die Bildgebungsvorrichtung 10e weist einen Überschaft 106e auf. Die Bildgebungskanäle 18e, 18'e sind gemeinsam in eine Überschaft 106e angeordnet. Der Überschaft 106e umfasst eine Mittelachse 108e. Die Bildgebungskanäle 18e, 18'e sind spiegelsymmetrisch zu dieser Mittelachse 108e angeordnet.

Die Bildgebungskanäle 18e, 18'e weisen jeweils wenigstens einen Bildgebungszweig 20e, 38e, 58e auf. Im vorliegenden Fall weisen die Bildgebungskanäle jeweils drei Bildgebungszweige 20e, 38e, 58e auf. Die Bildgebungskanäle 18e, 18'e sind wenigstens teilweise verschieden voneinander ausgebildet. Die Bildgebungskanäle unterscheiden sich wenigstens durch eine Spektralselektivität wenigstens eines spektralselektiven Strahlteilers 22e, 40e, 60e, 22'e, 40'e, 60'e ihrer Bildgebungszweige 20e, 38e, 58e, 20'e, 38'e, 58'e. Im vorliegenden Fall sind die Spektralselektivitäten aller weiterer spektralselektiven Strahlteiler 22'e, 40'e, 60'e des weiteren Bildgebungskanals 18'e von den Spektralselektivitäten aller spektralselektiven Strahlteiler 22e, 40e, 60e, des Bildgebungskanal 18e verschieden. Ferner unterscheiden sich die Bildgebungskanäle sich wenigstens durch eine Spektralempfindlichkeit 88e, 90e, 92e wenigstens eines Bildsensors 34e, 50e, 70e ihrer Bildgebungszweige 20e, 38e, 58e, 20'e, 38'e, 58'e. Im vorliegenden Fall sind die Spektralempfindlichkeiten 88e, 90e, 92e aller weiterer Bildsensoren 34'e, 50'e, 70'e des weiteren Bildgebungskanals 18'e von den Spektralempfindlichkeiten 88e, 90e, 92e aller spektralselektiven Strahlteiler 22e, 40e, 60e, des Bildgebungskanal 18e verschieden.

Bezüglich eins Verfahrens bietet diese Ausführung den Vorteil, dass mittels den unterschiedlichen Bildgebungszweigen 20e, 38e, 58e, 20'e, 38'e, 58'e ver-schiedene Gewebeeigenschaften und/oder Arten zugeordnet werden können. Um eine einheitliche Darstellung zu erhalten, können die verschiedenen Bilder z.B. durch Feature-Tracking gematcht werden, wodurch die vollständige Information auch für ein Stereobild zur Verfügung steht.

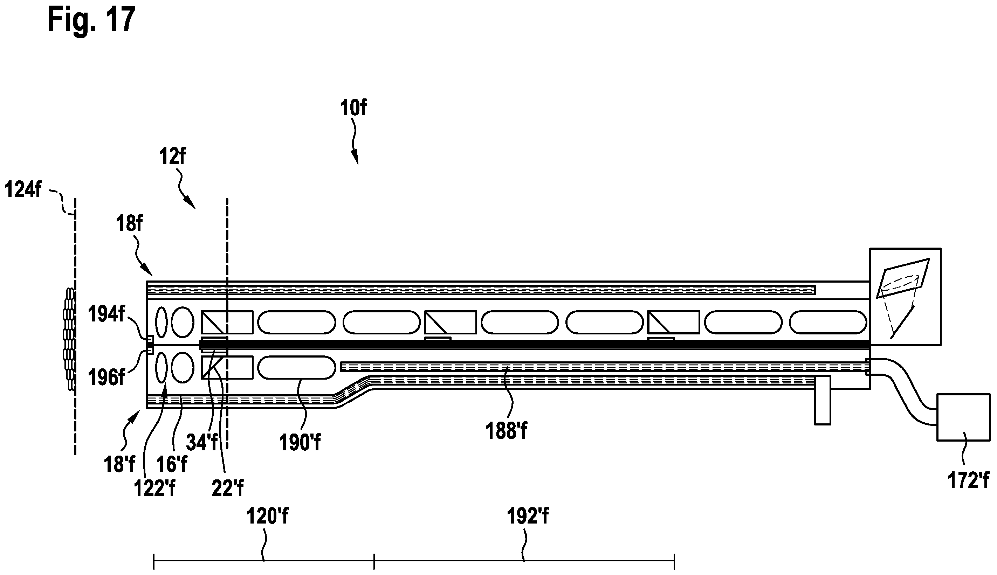

Fig. 17 zeigt eine schematische Darstellung einer weiteren alternativen endoskopischen und/oder exoskopischen Bildgebungsvorrichtung 10f in einer Schnittansicht. Die vorliegende Bildgebungsvorrichtung 10f unterscheidet sich von der vorhergehenden im Wesentlichen durch eine Ausgestaltung des weiteren Bildgebungskanals 18'f der Bildgebungsvorrichtung.

Der Bildgebungskanal 18f ist zu einer multispektralen bzw. hyperspektralen Bildgebung eingerichtet. Im Gegensatz dazu ist der weitere Bildgebungskanal 18'f ist zu einer Weißlichtbildgebung eingerichtet. Dadurch, dass die Bildgebungskanäle 18f, 18'f voneinander verschieden ausgebildet sind, z.B. durch eine unterschiedliche Anzahl von Strahlteiler, werden bei einer gemeinsamen Darstellung von mittels der Bildgebungskanälen aufgenommenen Abbildungen aufeinander abgestimmt, indem diese beispielsweise digital verstärkt und/oder Helligkeiten und/oder Kontraste angepasst werden.

Der weitere Bildgebungskanal 18'f weist ein Objektiv 122'f auf. Das Objektiv 122'f ist wenigstens teilweise in dem Schaft 16'f angeordnet. Das Objektiv 122'f ist im distalen Endabschnitt 120'f des Schafts 16'f angeordnet. Das Objektiv 122'f ist zur Erzeugung einer optischen Abbildung des Untersuchungsgebiets eingerichtet. Das Objektiv 122'f weist eine Objektebene 124'f auf. In einem Betriebszustand befindet sich das Untersuchungsgebiet in der Objektebene 124'f. Das Objektiv 122'f weist ferner eine Bildebene auf. Das Objektiv 122'f bildet eine optische Abbildung des sich in der Objektebene befindlichen Untersuchungsgebiet auf die Bildebene ab.

Der weitere Bildgebungskanal 18'f weist einen ersten Bildgebungszweig 20'f auf. Der weitere erste Bildgebungszweig 20'f ist wenigstens teilweise in dem Schaft 16' angeordnet. Im vorliegenden Fall ist der weitere erste Bildgebungszweig 20'f vollständig in dem Schaft 16'f angeordnet. Der weitere erste Bildgebungszweig 20'f ist dem Objektiv 122'f lichtstromaufwärts nachgeschaltet.

Der weitere erste Bildgebungszweig 20'f weist wenigstens einen ersten spektralselektiven Strahlteiler 22'f auf. Der erste spektralselektive Strahlteiler 22'f teilt die optische Abbildung des Untersuchungsgebiets in wenigstens eine erste spektrale Teilabbildung eines ersten Spektralbereichs und wenigstens eine weitere erste spektrale Teilabbildung eines weiteren ersten Spektralbereichs 32'f auf. Der erste spektralselektive Strahlteiler 22'f lenkt die erste spektrale Teilabbildung ab, im vorliegenden Fall um 90°. Die weitere erste spektrale Teilabbildung durchläuft den ersten spektralen Strahlteiler 22'f in gleichbleibender Richtung. Die erste spektrale Teilabbildung erstreckt sich über einen sichtbaren Spektralbereich. Die weitere erste spektrale Teilabbildung erstreckt sich über den nahen Infrarotbereich.

Der weitere erste Bildgebungszweig 20'f weist wenigstens einen weiteren ersten Bildsensor 34'f auf. Der weitere erste Bildsensor 34'f ist zur Erfassung der ersten spektralen Teilabbildung eingerichtet. Durch die Ablenkung des ersten spektralselektiven Strahlteilers 22'f ist die erste spektrale Teilabbildung auf den ersten Bildsensor gerichtet. Der erste Bildsensor 34'f liegt in einer Bildebene der ersten spektralen Teilabbildung. Somit erfolgt eine scharfe Abbildung der ersten spektralen Teilabbildung auf den ersten Bildsensor 34'f.

Der weitere erste Bildgebungszweig 20'f weist im vorliegenden Fall jedoch keine Relaisoptik auf. Somit ist auch der weitere Bildgebungskanal 18'f frei von einer Relaisoptik.

Stattdessen weist der Bildgebungskanal 18'f eine Lichtleitfaser 188'f auf. Die Lichtleitfaser 188'f ist in dem Schaft 16'f angeordnet. Zur Fokussierung der Teilabbildung auf die Lichtleitfaser 188'f umfasst der weitere erste Bildgebungszweig 20'f eine Fokuslinse 190'f. Die Fokuslinse 190'f ist zwischen Strahlteiler 22'f und Lichtleiterfaser 188'fangeordnet.

Ferner umfasst die Bildgebungsvorrichtung 10'f ein Spektrometer 172'f. Das Spektrometer 172'f ist dazu eingerichtet, ein Gesamtspektrum der weiteren ersten Teilabbildung aufzunehmen. Das Spektrometer 172'f ist mit der Lichtleitfaser verbunden.

Die Bildgebungsvorrichtung 10'f weist eine Steuereinrichtung auf. Die Steuereinrichtung ist dazu eingerichtet wenigstens ein mit dem Bildgebungskanal 18f aufgenommenes multispektrales und/oder hyperspektrales Bild mit wenigstens einem von dem weiteren Bildgebungskanal 18'f aufgenommenen Weißlichtbild zu matchen. Dazu nutzt die Steuereinrichtung einen Matching-Algorithmus. Im vorliegenden Fall wird Feature-Trackings verwendet, es kann aber auch ein anderer Algorithmus genutzt werden. Durch das Matchtig können sowohl Weißlicht als auch multispektrale bzw. hyperspektrale Stereobilder bzw. Stereovideos erzeugt werden, obwohl jeweils nur einer der Bildgebungskanäle18f, 18'f zu einer Weißlichtbildgebung bzw. Multispektral- und/oder Hyperspektralbildgebung eingerichtet ist.

Die Bildgebungsvorrichtung 10'f umfasst ferner wenigstens einen Temperatursensor 194f. In dem distalen Endabschnitt 120'f des Schafts 16'f ist der Temperatursensor 194f angeordnet. Der Temperatursensor 194f ist zu einer temperaturabhängigen Ansteuerung zumindest der Bildgebungskanäle 18f, 18'f eingerichtet. Durch gezieltes Aktivieren und/oder Deaktivieren der Elektronik, wie beispielsweise der Kamerabildsensoren kann ein Überschreiten einer Grenztemperatur vermieden werden. Ferner wird durch die einzuhaltende Grenztemperatur auch eine für eine Lichtquelle der Bildgebungsvorrichtung 10f einzuhaltende Bestrahlungszeit geregelt.

Die Bildgebungsvorrichtung 10'f weist wenigstens einen Bewegungssensor 196f auf. Der Bewegungssensor ist in einem distalen Endabschnitt 120'f des Schafts 16'f angeordnet. Der Bewegungssensor 196f ist zu einer bewegungsabhängigen Ansteuerung zumindest des Bildgebungskanäle 18f, 18'f eingerichtet ist. Der Bewegungssensor 196f ist dazu eingerichtet eine konstante Position der Bildgebungsvorrichtung 10'f während einer Aufnahme einer Abbildung zu registrieren. Für den Fall, dass eine Bewegung registriert wird, wird eine Meldung an die Steuereinrichtung übergeben und eine Aufnahme gestoppt. Ferner kann eine Bewegung mittels des Bewegungssensors 196f aufgezeichnet werden und eine Bildgebung basierend auf dieser erfassten Bewegung korrigiert werden.

Der Schaft 16'f weist einen distalen Endabschnitt 120'f auf, welcher gegenüber einem Mittelabschnitt 192'f verbreitert ist. In diesem distalen Endabschnitt 120'f sind sowohl der Bildgebungskanal 18'f als auch der weitere Bildgebungskanal 18'f angeordnet. Allerdings ist der Mittelabschnitt 192'f im Wesentlichen von dem Bildgebungskanal 18'f ausgefüllt.

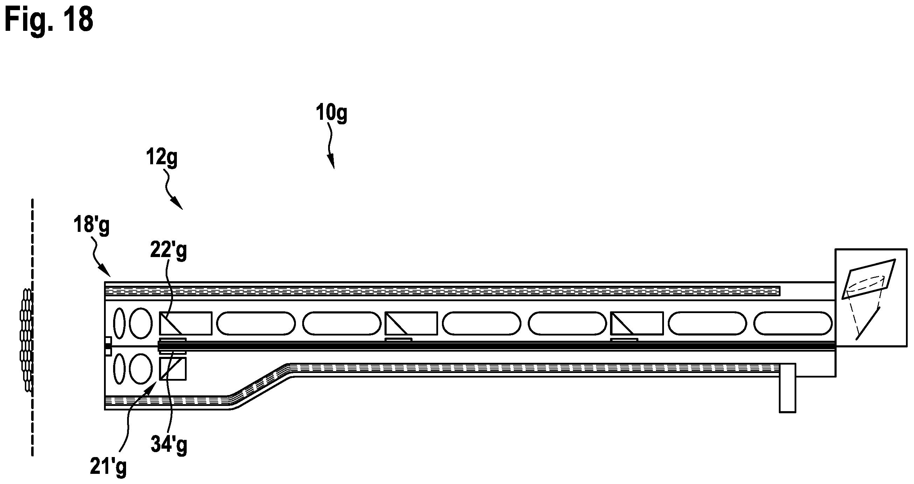

Fig. 18 zeigt eine schematische Darstellung einer zusätzlichen alternativen endoskopischen und/oder exoskopischen Bildgebungsvorrichtung 10g in einer Schnittansicht. Die vorliegende Bildgebungsvorrichtung 10g unterscheidet sich von der Vorhergehenden im Wesentlichen durch eine Ausgestaltung des weiteren Bildgebungskanals 18'g der Bildgebungsvorrichtung 10'g.

Im vorliegenden Fall weist der weitere Bildgebungskanal 18'g anstelle eines ersten Bildgebungszweigs einen ersten Bildgebungsstrang 21'g auf. Der Bildgebungsstrang 21'g ist im Wesentlichen äquivalent zu einem der vorher beschriebenen Bildgebungszweige ausgebildet, wobei dieser anstelle eines Strahlenteilers über einen Spiegel verfügt, welcher dazu eingerichtet ist Licht auf einen Bildsensor des Bildgebungsstrangs 21'g zu lenken. Der Bildgebungsstrang 21'f ist ferner frei von einer Relaisoptik. Ferner ist der Bildgebungsstrang 121'f frei von Abzweigungen eines optischen Pfads.

| | | | |
|---|---|---|---|
| 10 | Bildgebungsvorrichtung | 48 | Weitere zweiter Spektralbereich |
| 12 | Endoskop | 50 | Zweitere Bildsensor |
| 14 | Exoskop | 52 | Zweite Relaisoptik |
| 16 | Schaft | 54 | Bildebene der ersten Relaisoptik |
| 18 | Bildgebungskanal | | |
| 20 | Bildgebungszweig | 56 | Objektebene der zweiten Relaisoptik |
| 21 | Bildgebungsstrang | 58 | Dritter Bildgebungszweig |
| 22 | Spektralselektiver Strahlteiler | 60 | Dritter spektralselektiver Strahlteiler |
| 24 | Optische Abbildung | 62 | Dritte spektrale |
| 26 | Erste spektrale Teilabbildung | 64 | Teilabbildung Dritter Spektralbereich |
| 28 | Erste Spektralbereich | 66 | Weitere dritte spektrale |
| 30 | Weitere erste Teilabbildung | | Teilabbildung |
| 32 | Weiterer erster Spektralbereich | 68 | Weiterer dritter Spektralbereich |
| 34 | Erster Bildsensor | 70 | Dritter Bildsensor |
| 36 | Erste Relaisoptik | 72 | Dritte Relaisoptik |
| 38 | Zweite Bildgebungszweig | 74 | Bildebene der zweiten Relaisoptik |
| 40 | Zweitere spektralselektiver Strahlteiler | 76 | Objektebene der dritten Relaisoptik |
| 42 | Zweite spektrale Teilabbildung | 80 | Weiteres optisches Bauteil |
| 44 | Zweiter Spektralbereich | 82 | Sensorebene |
| 46 | Weitere zweite spektrale Teilabbildung | 84 | Mittelachse des Schafts |
| 86 | Leiterplatte | 128 | Objektebene der ersten Relaisoptik |
| 88 | Spektralempfindlichkeit | 130 | Stablinsenpaar |
| 90 | Spektralempfindlichkeit | 132 | Zweites weiteres optisches Bauteil |
| 92 | Spektralempfindlichkeit | | |
| 96 | Kamera | 134 | Bildebene der dritten Relaisoptik |
| 98 | Lichtquelle | 136 | Drittes weiteres optisches Bauteil |
| 100 | Beleuchtungsspektrum | | |
| 102 | Steuereinrichtung | 138 | Abzubildendes Untersuchungsgebiet |
| 104 | Leuchtelement | 140 | Beleuchtungskennlinie |
| 106 | Überschafft | 142 | Erste Spektralkennlinie |
| 108 | Mittelachse des Überschafts | 144 | Zweite Spektralkennlinie |
| 110 | Haupterstreckung des Überschafts | 146 | Dritte Spektralkennlinie |
| 112 | Distaler Endabschnitt des Überschafts | 148 | Vierte Spektralkennlinie |
| | | 150 | Erste Transmissionskennlinie |
| 114 | Proximaler Endabschnitt des Überschafts | | |
| | | 152 | zweite Transmissionskennlinie |
| 116 | Lichtwellenleiter | | |
| 118 | Haupterstreckung des Schafts | 154 | Dritte Transmissionskennlinie |
| 120 | Distaler Endabschnitt des Schafts | 156 | Erste Reflexionskennlinie |
| | | 158 | Zweite Reflexionskennlinie |
| 121 | Proximaler Endabschnitt des Schafts | 160 | Dritte Reflexionskennlinie |
| 122 | Objektiv | 162 | Erste Empfindlichkeitskennlinie |
| 124 | Objektebene des Objektivs | 164 | Zweite Empfindlichkeitskennlinie |
| 126 | Bildebene des Objektivs | | |
| 166 | Dritte Empfindlichkeitskennlinie | 186 | Verstelleinrichtung |
| 168 | Kameragehäuse | 188 | Lichtleitfase |
| 170 | Eingangsobjektiv | 190 | Fokuslinse |
| 172 | Spektrometer | 192 | Mittelabschnitt |
| 174 | Blende | 194 | Temperatursensor |
| 176 | Interne Optik | 196 | Bewegungssensor |
| 178 | Interne Linse | 200 | Verfahrensschritt |
| 180 | Dispersives Element | 202 | Verfahrensschritt |
| 182 | Weitere interne Linse | 204 | Verfahrensschritt |
| 184 | Kamerabildsensor | 206 | Verfahrensschritt |

## Patentansprüche

1. Endoskopische- und/oder exoskopische Bildgebungsvorrichtung (10) zur spektralen, insbesondere multispektralen und/oder hyperspektralen Bildgebung für ein Endoskop (12) und/oder ein Exoskop (14) mit wenigstens einem Schaft (16) und mit wenigstens einem zumindest teilweise in dem Schaft (16) angeordneten Bildgebungskanal (18), welcher wenigstens einen ersten Bildgebungszweig (20) aufweist, welcher wenigstens einen ersten spektralselektiven Strahlteiler (22) umfasst, der spektralselektiv eine optische Abbildung (24) eines ursprünglichen Spektralbereichs (28, 32, 44, 48, 64, 68) in wenigstens eine erste spektrale Teilabbildung (26) eines ersten Spektralbereichs (28) und wenigstens eine weitere erste spektrale Teilabbildung (30) eines weiteren ersten Spektralbereichs (32) aufteilt, wobei der erste Spektralbereich (28) von dem weiteren ersten Spektralbereich (32) verschieden ist, und der erste Bildgebungszweig (20) wenigstens zur Erfassung der ersten spektralen Teilabbildung (26) einen ersten Bildsensor (34) umfasst, sowie der erste Bildgebungszweig (20) zur Weiterleitung der weiteren ersten Teilabbildung (30) wenigstens eine erste Relaisoptik (36) umfasst, die ein Stablinsenpaar (130) aufweist, **dadurch gekennzeichnet, dass** der Bildgebungskanal (18) wenigstens einen zweiten Bildgebungszweig (38) aufweist, welcher wenigstens einen zweiten spektralselektiven Strahlteiler (40) umfasst, der spektralselektiv die weitere erste Teilabbildung (30) des weiteren ersten Spektralbereichs (32) in wenigstens eine zweite spektrale Teilabbildung (42) eines zweiten Spektralbereichs (44) und wenigstens eine weitere zweite spektrale Teilabbildung (46) eines weiteren zweiten Spektralbereichs (48) aufteilt, wobei der zweite Spektralbereich (44) von dem weiteren zweiten Spektralbereich (48) verschieden ist, und der zweite Bildgebungszweig (38) wenigstens zur Erfassung der zweiten spektralen Teilabbildung (42) wenigstens einen zweiten Bildsensor (50) umfasst, sowie der zweite Bildgebungszweig (38) zur Weiterleitung der weiteren zweiten Teilabbildung (46) wenigstens eine zweite Relaisoptik (52) umfasst, die ein Stablinsenpaar (130) aufweist, wobei die erste Relaisoptik (36) des ersten Bildgebungszweigs (20) und die zweite Relaisoptik (52) des zweiten Bildgebungszweigs (38) hintereinander angeordnet sind, so dass eine Bildebenen (54) der ersten Relaisoptik (36) mit einer Objektebene (56) der zweiten Relaisoptik (52) identisch ist.

2. Bildgebungsvorrichtung (10) nach Anspruch 1, **dadurch gekennzeichnet, dass** der Bildgebungskanal (18) wenigstens einen dritten Bildgebungszweig (58) aufweist, welcher wenigstens einen dritten spektralselektiven Strahlteiler (60) umfasst, der spektralselektiv die weitere zweite spektrale Teilabbildung (46) des weiteren zweiten Spektralbereichs in wenigstens eine dritte spektrale Teilabbildung (62) eines dritten Spektralbereichs (64) und wenigstens eine weitere dritte spektrale Teilabbildung (66) eines weiteren dritten Spektralbereichs (68) aufteilt, wobei der dritte Spektralbereich (64) von dem weiteren dritten Spektralbereich (68) verschieden ist, und der dritte Bildgebungszweig (58) wenigstens zur Erfassung der dritten spektralen Teilabbildung (62) wenigstens einen dritten Bildsensor (70) umfasst, sowie der dritte Bildgebungszweig (58) zur Weiterleitung der weiteren dritten spektralen Teilabbildung (66) wenigstens eine dritte Relaisoptik (72) umfasst, wobei die zweite Relaisoptik (52) des zweiten Bildgebungszweigs (38) und die dritte Relaisoptik (72) des dritten Bildgebungszweigs (58) hintereinander angeordnet sind, so dass eine Bildebene (74) der zweiten Relaisoptik (52) mit einer Objektebene (76) der dritten Relaisoptik (72) identisch ist.

3. Bildgebungsvorrichtung (10) nach Anspruch 1 oder 2, **dadurch gekennzeichnet, dass** wenigstens ein spektralselektiver Strahlteiler (22, 40, 60) und/oder wenigstens ein Bildsensor (34, 50, 70) der Bildgebungszweige (20, 38, 58) zwischen zwei Relaisoptiken (36, 52, 72) verschiedener Bildgebungszweige (20, 38, 58) angeordnet ist.

4. Bildgebungsvorrichtung (10) nach einem der vorhergehenden Ansprüche, **dadurch gekennzeichnet, dass** wenigstens einer der Bildgebungszweige (20, 38, 58) wenigstens ein weiteres optisches Bauteil (80) umfasst, dessen Brechungsindex und/oder dessen Material zumindest im Wesentlichen mit einem Brechungsindex und/oder einem Material eines spektralselektiven Strahlteilers (22, 40, 60) des jeweiligen Bildgebungszweigs (20, 38, 58) übereinstimmt, und zwischen zwei Relaisoptiken (36, 52, 72) verschiedener Bildgebungszweige (20, 38, 58) angeordnet ist.

5. Bildgebungsvorrichtung (10) nach einem der vorhergehenden Ansprüche, **dadurch gekennzeichnet, dass** wenigstens einer der Spektralbereiche (28, 32, 44, 48, 64, 68) der Teilabbildungen (26, 30, 42, 46, 62, 66) der Bildgebungszweige (20, 38, 58) kürzere Wellenlängen umfasst, als wenigstens ein anderer Spektralbereich (28, 32, 44, 48, 64, 68) der Teilabbildungen (26, 30, 42, 46, 62, 66) der Bildgebungszweige (20, 38, 58).

6. Bildgebungsvorrichtung (10) nach einem der vorhergehenden Ansprüche, **dadurch gekennzeichnet, dass** zumindest ein Bildsensor (34, 50, 70) der Bildgebungszweige (20, 38, 58) wenigstens eine Spektralempfindlichkeit (88, 90, 92) aufweist, welche von einer Spektralempfindlichkeit (88, 90, 92) eines anderen Bildsensors (34, 50, 70) der Bildgebungszweige (20, 38, 58) verschieden ist.

7. Bildgebungsvorrichtung (10) nach einem der vorhergehenden Ansprüche, **dadurch gekennzeichnet, dass** wenigstens ein Bildsensor (34, 50, 70) oder wenigstens zwei Bildsensoren (34, 50, 70) der Bildgebungszweige (20, 38, 58) wenigstens eine erste Spektralempfindlichkeit (88) und wenigstens eine zweite Spektralempfindlichkeit (90), sowie insbesondere wenigstens eine dritte Spektralempfindlichkeit (92), aufweist/aufweisen, in Abhängigkeit welcher dieser/diese eine Teilabbildung (26, 30, 42, 46, 62, 66) der Bildgebungszweige (20, 38, 58) erfasst.

8. Bildgebungsvorrichtung (10) nach einem der vorhergehenden Ansprüche, **dadurch gekennzeichnet, dass** der Bildergebungskanal (18) zur Erfassung wenigstens einer der Teilabbildungen (26, 30, 42, 46, 62, 66) der Bildgebungszweige (20, 38, 58) wenigstens eine Kamera (96) aufweist, welche an einem proximalen und insbesondere extrakorporalen Endabschnitts (121) des Schafts (16) angeordnet ist und vorzugsweise als eine multispektrale und/oder hyperspektrale Kamera ausgebildet ist.

9. Bildgebungsvorrichtung (10) nach einem der vorhergehenden Ansprüche, **gekennzeichnet durch** wenigstens eine Lichtquelle (98), welche in wenigstens einem Betriebszustand ein abzubildendes Untersuchungsgebiet mit wenigstens einem Beleuchtungsspektrum (100) beleuchtet.

10. Bildgebungsvorrichtung (10) nach Anspruch 9, **dadurch gekennzeichnet, dass** die Lichtquelle (98) Beleuchtungslicht abstrahlt, welches ein Beleuchtungsspektrum (100) umfasst, welches breiter ist, als wenigstens ein Spektralbereich (28, 32, 44, 48, 64, 68) der Teilabbildungen (26, 30, 42, 46, 62, 66) der Bildgebungszweige (20, 38, 58) und/oder die Lichtquelle (98) Beleuchtungslicht abstrahlt, welches wenigstens ein Beleuchtungsspektrum (100) umfasst, welches innerhalb wenigstens eines Spektralbereichs (28, 32, 44, 48, 64, 68) der Teilabbildungen (26, 30, 42, 46, 62, 66) der Bildgebungszweige (20, 38, 58) liegt.

11. Bildgebungsvorrichtung (10) nach einem der vorhergehenden Ansprüche, **gekennzeichnet durch** wenigstens einen weiteren Bildgebungskanal (18'), wobei insbesondere der Bildgebungskanal (18) und der weitere Bildgebungskanal (18') zueinander spiegelsymmetrisch angeordnet sind.

12. Bildgebungsvorrichtung (10) nach Anspruch 11, **dadurch gekennzeichnet, dass** sich die Bildkanäle (18, 18') wenigstens durch eine Spektralselektivität wenigstens eines spektralselektiven Strahlteilers (22, 40, 60) ihrer Bildgebungszweige (20, 38, 58) und/oder durch eine Spektralempfindlichkeit 88, 90, 92 wenigstens eines Bildsensors (34, 50, 70) ihrer Bildgebungszweige (20, 38, 58) voneinander unterscheiden.

13. Bildgebungsvorrichtung (10) nach einem der Ansprüche 1 bis 12, **gekennzeichnet durch** wenigstens einen weiteren Bildgebungskanal (18'), welcher frei von einer Relaisoptik ist, wobei insbesondere der Bildgebungskanal (18) zu einer multispektralen und/oder hyperspektralen Bildgebung eingerichtet ist und der weitere Bildgebungskanal (18') zu einer Weißlichtbildgebung eingerichtet ist.

14. Bildgebungsvorrichtung (10) nach Anspruch 13, **gekennzeichnet durch** eine Steuereinrichtung (102), welche dazu eingerichtet ist, wenigstens ein mit dem Bildgebungskanal (18) aufgenommenes multispektrales und/oder hyperspektrales Bild mit wenigstens einem von dem weiteren Bildgebungskanal (18') aufgenommenen Weißlichtbild zu matchen.

15. Bildgebungsvorrichtung (10) nach einem der vorhergehenden Ansprüche, **dadurch gekennzeichnet, dass** in einem distalen Endabschnitt (120) des Schafts (16) wenigstens ein Temperatursensor angeordnet ist, welcher zu einer temperaturabhängigen Ansteuerung zumindest des Bildgebungskanals (18) eingerichtet ist und/oder in einem distalen Endabschnitt (120) des Schafts (16) wenigstens ein Bewegungssensor angeordnet ist, welcher zu einer bewegungsabhängigen Ansteuerung zumindest des Bildgebungskanals (18) eingerichtet ist.

16. Endoskop (12) und/oder Exoskop (14) mit wenigstens einer Bildgebungsvorrichtung (10) nach einem der vorhergehenden Ansprüche.

## Claims

1. Endoscopic and/or exoscopic imaging apparatus (10) for spectral, in particular multispectral and/or hyperspectral, imaging for an endoscope (12) and/or an exoscope (14), having at least one shaft (16) and having at least one imaging channel (18) arranged at least partially in the shaft (16), which imaging channel has at least a first imaging branch (20) comprising at least a first spectrally selective beam splitter (22) which spectrally selectively divides an optical image (24) of an original spectral range (28, 32, 44, 48, 64, 68) into at least one first spectral partial image (26) of a first spectral range (28) and at least one further first spectral partial image (30) of a further first spectral range (32), the first spectral range (28) being different from the further first spectral range (32), and the first imaging branch (20) comprising a first image sensor (34) at least for capturing the first spectral partial image (26), and the first imaging branch (20) comprising at least a first relay optic (36) for transmitting the further first partial image (30), which relay optic has a rod lens pair (130), **characterized in that** the imaging channel (18) has at least a second imaging branch (38) comprising at least a second spectrally selective beam splitter (40) which spectrally selectively divides the further first partial image (30) of the further first spectral range (32) into at least one second spectral partial image (42) of a second spectral range (44) and at least one further second spectral partial image (46) of a further second spectral range (48), the second spectral range (44) being different from the further second spectral range (48), and the second imaging branch (38) comprising at least a second image sensor (50) at least for capturing the second spectral partial image (42), and the second imaging branch (38) comprising at least a second relay optic (52) for transmitting the further second partial image (46), which relay optic has a rod lens pair (130), the first relay optic (36) of the first imaging branch (20) and the second relay optic (52) of the second imaging branch (38) being arranged one behind the other, such that an image plane (54) of the first relay optic (36) is identical to an object plane (56) of the second relay optic (52).

2. Imaging apparatus (10) according to claim 1, **characterized in that** the imaging channel (18) has at least a third imaging branch (58) comprising at least a third spectrally selective beam splitter (60) which spectrally selectively divides the further second spectral partial image (46) of the further second spectral range into at least one third spectral partial image (62) of a third spectral range (64) and at least one further third spectral partial image (66) of a further third spectral range (68), the third spectral range (64) being different from the further third spectral range (68), and the third imaging branch (58) comprising at least a third image sensor (70) at least for capturing the third spectral partial image (62), and the third imaging branch (58) comprising at least a third relay optic (72) for transmitting the further third spectral partial image (66), the second relay optic (52) of the second imaging branch (38) and the third relay optic (72) of the third imaging branch (58) being arranged one behind the other, such that an image plane (74) of the second relay optic (52) is identical to an object plane (76) of the third relay optic (72).

3. Imaging apparatus (10) according to claim 1 or 2, **characterized in that** at least one spectrally selective beam splitter (22, 40, 60) and/or at least one image sensor (34, 50, 70) of the imaging branches (20, 38, 58) is arranged between two relay optics (36, 52, 72) of different imaging branches (20, 38, 58).

4. Imaging apparatus (10) according to any of the preceding claims, **characterized in that** at least one of the imaging branches (20, 38, 58) comprises at least one further optical component (80), the refractive index and/or material of which at least substantially matches a refractive index and/or a material of a spectrally selective beam splitter (22, 40, 60) of the corresponding imaging branch (20, 38, 58), and is arranged between two relay optics (36, 52, 72) of different imaging branches (20, 38, 58).

5. Imaging apparatus (10) according to any of the preceding claims, **characterized in that** at least one of the spectral ranges (28, 32, 44, 48, 64, 68) of the partial images (26, 30, 42, 46, 62, 66) of the imaging branches (20, 38, 58) encompasses shorter wavelengths than at least one other spectral range (28, 32, 44, 48, 64, 68) of the partial images (26, 30, 42, 46, 62, 66) of the imaging branches (20, 38, 58).

6. Imaging apparatus (10) according to any of the preceding claims, **characterized in that** at least one image sensor (34, 50, 70) of the imaging branches (20, 38, 58) has at least one spectral sensitivity (88, 90, 92) which is different from a spectral sensitivity (88, 90, 92) of another image sensor (34, 50, 70) of the imaging branches (20, 38, 58).

7. Imaging apparatus (10) according to any of the preceding claims, **characterized in that** at least one image sensor (34, 50, 70) or at least two image sensors (34, 50, 70) of the imaging branches (20, 38, 58) has/have at least a first spectral sensitivity (88) and at least a second spectral sensitivity (90), and in particular at least a third spectral sensitivity (92), depending on which this image sensor/these image sensors captures a partial image (26, 30, 42, 46, 62, 66) of the imaging branches (20, 38, 58).

8. Imaging apparatus (10) according to any of the preceding claims, **characterized in that** the imaging channel (18) for capturing at least one of the partial images (26, 30, 42, 46, 62, 66) of the imaging branches (20, 38, 58) has at least one camera (96) which is arranged on a proximal and in particular extracorporeal end portion (121) of the shaft (16) and is preferably in the form of a multispectral and/or hyperspectral camera.

9. Imaging apparatus (10) according to any of the preceding claims, **characterized by** at least one light source (98) which illuminates a region subject to investigation that is to be imaged with at least one illumination spectrum (100) in at least one operating state.

10. Imaging apparatus (10) according to claim 9, **characterized in that** the light source (98) emits illuminating light which encompasses an illumination spectrum (100) which is wider than at least one spectral range (28, 32, 44, 48, 64, 68) of the partial images (26, 30, 42, 46, 62, 66) of the imaging branches (20, 38, 58) and/or the light source (98) emits illuminating light which encompasses at least one illumination spectrum (100) which lies within at least one spectral range (28, 32, 44, 48, 64, 68) of the partial images (26, 30, 42, 46, 62, 66) of the imaging branches (20, 38, 58).

11. Imaging apparatus (10) according to any of the preceding claims, **characterized by** at least one further imaging channel (18'), in particular the imaging channel (18) and the further imaging channel (18') being arranged in a mirror-symmetrical manner relative to each other.

12. Imaging apparatus (10) according to claim 11, **characterized in that** the image channels (18, 18') differ from each other at least by a spectral selectivity of at least one spectrally selective beam splitter (22, 40, 60) of their imaging branches (20, 38, 58) and/or by a spectral sensitivity 88, 90, 92 of at least one image sensor (34, 50, 70) of their imaging branches (20, 38, 58).

13. Imaging apparatus (10) according to any of claims 1 to 12, **characterized by** at least one further imaging channel (18') which is free of a relay optic, in particular the imaging channel (18) being configured for multispectral and/or hyperspectral imaging and the further imaging channel (18') being configured for white light imaging.

14. Imaging apparatus (10) according to claim 13, **characterized by** a control device (102) which is configured to match at least one multispectral and/or hyperspectral image received by the imaging channel (18) with at least one white light image received by the further imaging channel (18').

15. Imaging apparatus (10) according to any of the preceding claims, **characterized in that** at least one temperature sensor is arranged in a distal end portion (120) of the shaft (16), which temperature sensor is configured for temperature-dependent control of at least the imaging channel (18) and/or at least one motion sensor is arranged in a distal end portion (120) of the shaft (16), which motion sensor is configured for motion-dependent control of at least the imaging channel (18).

16. Endoscope (12) and/or exoscope (14) comprising at least one imaging apparatus (10) according to any of the preceding claims.

## Revendications

1. Dispositif d'imagerie (10) endoscopique et/ou exoscopique pour l'imagerie spectrale, en particulier multispectrale et/ou hyperspectrale, pour un endoscope (12) et/ou un exoscope (14), comportant au moins une tige (16) et comportant au moins un canal d'imagerie (18) disposé au moins partiellement dans la tige (16), lequel canal d'imagerie présente au moins une première branche d'imagerie (20) qui comprend au moins un premier séparateur de faisceaux (22) sélectif en termes de spectre, lequel sépare de manière sélective en termes de spectre une représentation optique (24) d'un domaine spectral initial (28, 32, 44, 48, 64, 68) en au moins une première représentation partielle spectrale (26) d'un premier domaine spectral (28) et en au moins une autre première représentation partielle spectrale (30) d'un autre premier domaine spectral (32), dans lequel le premier domaine spectral (28) est différent de l'autre premier domaine spectral (32), et la première branche d'imagerie (20) comprend, au moins pour la détection de la première représentation partielle spectrale (26), un premier capteur d'image (34), et la première branche d'imagerie (20) comprend, pour la transmission de l'autre première représentation partielle (30), au moins une première optique de relais (36) qui présente une paire de lentilles barreaux (130), **caractérisé en ce que** le canal d'imagerie (18) présente au moins une deuxième branche d'imagerie (38) qui comprend au moins un deuxième séparateur de faisceaux (40) sélectif en termes de spectre, lequel sépare de manière sélective en termes de spectre l'autre première représentation partielle (30) de l'autre premier domaine spectral (32) en au moins une deuxième représentation partielle spectrale (42) d'un deuxième domaine spectral (44) et en au moins une autre deuxième représentation partielle spectrale (46) d'un autre deuxième domaine spectral (48), dans lequel le deuxième domaine spectral (44) est différent de l'autre deuxième domaine spectral (48), et la deuxième branche d'imagerie (38) comprend, au moins pour la détection de la deuxième représentation partielle spectrale (42), au moins un deuxième capteur d'image (50), et la deuxième branche d'imagerie (38) comprend, pour la transmission de l'autre deuxième représentation partielle (46), au moins une deuxième optique de relais (52) qui présente une paire de lentilles barreaux (130), dans lequel la première optique de relais (36) de la première branche d'imagerie (20) et la deuxième optique de relais (52) de la deuxième branche d'imagerie (38) sont disposées l'une derrière l'autre, de sorte qu'un plan d'image (54) de la première optique de relais (36) est identique à un plan d'objet (56) de la deuxième optique de relais (52).

2. Dispositif d'imagerie (10) selon la revendication 1, **caractérisé en ce que** le canal d'imagerie (18) présente au moins une troisième branche d'imagerie (58) qui comprend au moins un troisième séparateur de faisceaux (60) sélectif en termes de spectre, lequel sépare de manière sélective en termes de spectre l'autre deuxième représentation partielle spectrale (46) de l'autre deuxième domaine spectral en au moins une troisième représentation partielle spectrale (62) d'un troisième domaine spectral (64) et en au moins une autre troisième représentation partielle spectrale (66) d'un autre troisième domaine spectral (68), dans lequel le troisième domaine spectral (64) est différent de l'autre troisième domaine spectral (68), et la troisième branche d'imagerie (58) comprend, au moins pour la détection de la troisième représentation partielle spectrale (62), au moins un troisième capteur d'image (70), et la troisième branche d'imagerie (58) comprend, pour la transmission de l'autre troisième représentation partielle spectrale (66), au moins une troisième optique de relais (72), dans lequel la deuxième optique de relais (52) de la deuxième branche d'imagerie (38) et la troisième optique de relais (72) de la troisième branche d'imagerie (58) sont disposées l'une derrière l'autre, de sorte qu'un plan d'image (74) de la deuxième optique de relais (52) est identique à un plan d'objet (76) de la troisième optique de relais (72).

3. Dispositif d'imagerie (10) selon la revendication 1 ou 2, **caractérisé en ce qu'**au moins un séparateur de faisceaux (22, 40, 60) sélectif en termes de spectre et/ou au moins un capteur d'image (34, 50, 70) des branches d'imagerie (20, 38, 58) sont disposés entre deux optiques de relais (36, 52, 72) de différentes branches d'imagerie (20, 38, 58).

4. Dispositif d'imagerie (10) selon l'une des revendications précédentes, **caractérisé en ce qu'**au moins l'une des branches d'imagerie (20, 38, 58) comprend au moins un autre composant optique (80) dont l'indice de réfraction et/ou le matériau coïncident au moins sensiblement avec un indice de réfraction et/ou un matériau d'un séparateur de faisceaux (22, 40, 60) sélectif en termes de spectre de la branche d'imagerie (20, 38, 58) respective, et qui est disposé entre deux optiques de relais (36, 52, 72) de différentes branches d'imagerie (20, 38, 58).

5. Dispositif d'imagerie (10) selon l'une des revendications précédentes, **caractérisé en ce qu'**au moins l'un des domaines spectraux (28, 32, 44, 48, 64, 68) des représentations partielles (26, 30, 42, 46, 62, 66) des branches d'imagerie (20, 38, 58) comprend des longueurs d'onde plus courtes que celles d'au moins un autre domaine spectral (28, 32, 44, 48, 64, 68) des représentations partielles (26, 30, 42, 46, 62, 66) des branches d'imagerie (20, 38, 58).

6. Dispositif d'imagerie (10) selon l'une des revendications précédentes, **caractérisé en ce qu'**au moins un capteur d'image (34, 50, 70) des branches d'imagerie (20, 38, 58) présente au moins une sensibilité spectrale (88, 90, 92) qui est différente d'une sensibilité spectrale (88, 90, 92) d'un autre capteur d'image (34, 50, 70) des branches d'imagerie (20, 38, 58).

7. Dispositif d'imagerie (10) selon l'une des revendications précédentes, **caractérisé en ce qu'**au moins un capteur d'image (34, 50, 70) ou au moins deux capteurs d'image (34, 50, 70) des branches d'imagerie (20, 38, 58) présentent au moins une première sensibilité spectrale (88) et au moins une deuxième sensibilité spectrale (90), ainsi que, en particulier, au moins une troisième sensibilité spectrale (92), en fonction desquelles ceux-ci détectent une représentation partielle (26, 30, 42, 46, 62, 66) des branches d'imagerie (20, 38, 58).

8. Dispositif d'imagerie (10) selon l'une des revendications précédentes, **caractérisé en ce que** le canal d'imagerie (18), pour la détection d'au moins l'une des représentations partielles (26, 30, 42, 46, 62, 66) des branches d'imagerie (20, 38, 58), présente au moins une caméra (96) qui est disposée sur une section d'extrémité proximale et en particulier extracorporelle (121) de la tige (16) et qui est de préférence réalisée comme une caméra multispectrale et/ou hyperspectrale.

9. Dispositif d'imagerie (10) selon l'une des revendications précédentes, **caractérisé par** au moins une source lumineuse (98) qui, dans au moins un état de fonctionnement, éclaire une zone d'examen à représenter avec au moins un spectre d'éclairage (100).

10. Dispositif d'imagerie (10) selon la revendication 9, **caractérisé en ce que** la source lumineuse (98) émet une lumière d'éclairage qui comprend un spectre d'éclairage (100) qui est plus large qu'au moins un domaine spectral (28, 32, 44, 48, 64, 68) des représentations partielles (26, 30, 42, 46, 62, 66) des branches d'imagerie (20, 38, 58) et/ou la source lumineuse (98) émet une lumière d'éclairage qui comprend au moins un spectre d'éclairage (100) qui se situe à l'intérieur d'au moins un domaine spectral (28, 32, 44, 48, 64, 68) des représentations partielles (26, 30, 42, 46, 62, 66) des branches d'imagerie (20, 38, 58).

11. Dispositif d'imagerie (10) selon l'une des revendications précédentes, **caractérisé par** au moins un autre canal d'imagerie (18'), dans lequel le canal d'imagerie (18) et l'autre canal d'imagerie (18') sont en particulier disposés avec une symétrie en miroir l'un par rapport à l'autre.

12. Dispositif d'imagerie (10) selon la revendication 11,
**caractérisé en ce que** les canaux d'image (18, 18') se distinguent les uns des autres au moins par une sélectivité spectrale d'au moins un séparateur de faisceaux (22, 40, 60) sélectif en termes de spectre de leurs branches d'imagerie (20, 38, 58) et/ou par une sensibilité spectrale (88, 90, 92) d'au moins un capteur d'image (34, 50, 70) de leurs branches d'imagerie (20, 38, 58).

13. Dispositif d'imagerie (10) selon l'une des revendications 1 à 12, **caractérisé par** au moins un autre canal d'imagerie (18') qui est exempt d'une optique de relais, dans lequel le canal d'imagerie (18) est en particulier configuré pour une imagerie multispectrale et/ou hyperspectrale et l'autre canal d'imagerie (18') est configuré pour une imagerie en lumière blanche.

14. Dispositif d'imagerie (10) selon la revendication 13, **caractérisé par** un appareil de commande (102) qui est configuré pour mettre en correspondance au moins une image multispectrale et/ou hyperspectrale prise par le canal d'imagerie (18) avec au moins une image en lumière blanche prise par l'autre canal d'imagerie (18').

15. Dispositif d'imagerie (10) selon l'une des revendications précédentes, **caractérisé en ce qu'**est disposé dans une section d'extrémité distale (120) de la tige (16) au moins un capteur de température qui est configuré pour une commande en fonction de la température d'au moins le canal d'imagerie (18) et/ou est disposé dans une section d'extrémité distale (120) de la tige (16) au moins un capteur de mouvement qui est configuré pour une commande en fonction du mouvement d'au moins le canal d'imagerie (18).

16. Endoscope (12) et/ou exoscope (14) comportant au moins un dispositif d'imagerie (10) selon l'une des revendications précédentes.
